(19) Europäisches Patentamt / European Patent Office / Office européen des brevets

(11) EP 3 359 682 B1

(12) # EUROPEAN PATENT SPECIFICATION

(45) Date of publication and mention of the grant of the patent: **11.11.2020 Bulletin 2020/46**

(21) Application number: **16770700.9**

(22) Date of filing: **09.09.2016**

(51) Int Cl.: ***C12Q 1/6883*** *(2018.01)* ***C12Q 1/689*** *(2018.01)*

(86) International application number: **PCT/EP2016/071351**

(87) International publication number: **WO 2017/060039 (13.04.2017 Gazette 2017/15)**

(54) **METHOD FOR DIAGNOSING HEPATIC FIBROSIS BASED ON BACTERIAL PROFILE AND DIVERSITY**

VERFAHREN ZUR DIAGNOSE VON LEBERFIBROSE AUF BASIS VON BAKTERIELLEM PROFIL UND DIVERSITÄT

PROCÉDÉ POUR DIAGNOSTIQUER UNE FIBROSE HÉPATIQUE SUR LA BASE DU PROFIL BACTÉRIEN ET LA DIVERSITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2015 EP 15306606**

(43) Date of publication of application: **15.08.2018 Bulletin 2018/33**

(73) Proprietors:
- **Vaiomer**
  **31670 Labège (FR)**
- **Universita' degli Studi di Roma " Tor Vergata"**
  **00173 Roma (IT)**
- **Fundació Institut d'Investigació Biomèdica de Girona Dr. Josep Trueta**
  **17017 Girona (ES)**

(72) Inventors:
- **LELOUVIER, Benjamin**
  **31400 Toulouse (FR)**
- **SERVANT, Florence**
  **31450 Belberaud (FR)**
- **COURTNEY, Michael**
  **26790 Suze La Rousse (FR)**
- **FEDERICI, Massimo**
  **00136 Roma (IT)**
- **FERNANDEZ-REAL, Jose Manuel**
  **17003 Gerone (ES)**

(74) Representative: **Lavoix**
**2, place d'Estienne d'Orves**
**75441 Paris Cedex 09 (FR)**

(56) References cited:
**WO-A1-2014/107619** **WO-A1-2015/162200**
**UA-U- 77 726** **UA-U- 77 798**

- **GAKUHEI SON ET AL: "Contribution of Gut Bacteria to Liver Pathobiology", GASTROENTEROLOGY RESEARCH AND PRACTICE, vol. 44, no. 16, 1 January 2010 (2010-01-01), pages 1193-13, XP055256089, ISSN: 1687-6121, DOI: 10.1073/pnas.0807390106**
- **A WILLEMS ET AL: "Comamonadaceae, a New Family Encompassing the Acidovorans rRNA Complex, Including Variovorax paradoxus gen. nov., comb. nov., for Alcaligenes paradoxus (Davis 1969)", INTERNATIONAL JOURNAL OF SYSTEMATIC BACTERIOLOGY, vol. 41, no. 3, 1 July 1991 (1991-07-01), pages 445-450, XP055257520, GB ISSN: 0020-7713, DOI: 10.1099/00207713-41-3-445**
- **DERRICK E FOUTS ET AL: "Bacterial translocation and changes in the intestinal microbiome in mouse models of liver disease", JOURNAL OF HEPATOLOGY, ELSEVIER, AMSTERDAM, NL, vol. 56, no. 6, 3 January 2012 (2012-01-03), pages 1283-1292, XP028487676, ISSN: 0168-8278, DOI: 10.1016/J.JHEP.2012.01.019 [retrieved on 2012-02-09]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- THOMAS FLASS ET AL: "Intestinal Lesions Are Associated with Altered Intestinal Microbiome and Are More Frequent in Children and Young Adults with Cystic Fibrosis and Cirrhosis", PLOS ONE, vol. 10, no. 2, 6 February 2015 (2015-02-06), page e0116967, XP055256097, DOI: 10.1371/journal.pone.0116967
- DAE WON JUN ET AL: "Association Between Small Intestinal Bacterial Overgrowth and Peripheral Bacterial DNA in Cirrhotic Patients", DIGESTIVE DISEASES AND SCIENCES, KLUWER ACADEMIC PUBLISHERS-PLENUM PUBLISHERS, NE, vol. 55, no. 5, 11 June 2009 (2009-06-11), pages 1465-1471, XP019816559, ISSN: 1573-2568
- AQEL BASHAR ET AL: "Role of the Gut Microbiome in Nonalcoholic Fatty Liver Disease", NUTRITION IN CLINICAL PRACTICE : NCP ; AN OFF. PUBL. OF THE AMERICAN SOCIETY FOR PARENTERAL AND ENTERAL NUTR,, vol. 30, no. 6, 8 October 2015 (2015-10-08), - 8 October 2015 (2015-10-08), pages 780-786, XP009188956, ISSN: 1941-2452, DOI: 10.1177/0884533615605811
- JEAN-MARC SABATÉ ET AL: "High Prevalence of Small Intestinal Bacterial Overgrowth in Patients with Morbid Obesity: A Contributor to Severe Hepatic Steatosis", OBESITY SURGERY, vol. 18, no. 4, 20 February 2008 (2008-02-20), pages 371-377, XP055143104, ISSN: 0960-8923, DOI: 10.1007/s11695-007-9398-2
- MORGAN G I LANGILLE ET AL: "Predictive functional profiling of microbial communities using 16S rRNA marker gene sequences", NATURE BIOTECHNOLOGY, vol. 31, no. 9, 25 August 2013 (2013-08-25), pages 814-821, XP055328402, US ISSN: 1087-0156, DOI: 10.1038/nbt.2676
- BENJAMIN LELOUVIER ET AL: "Changes in blood microbiota profiles associated with liver fibrosis in obese patients: A pilot analysis", HEPATOLOGY, vol. 64, no. 6, 18 November 2016 (2016-11-18), pages 2015-2027, XP055328057, ISSN: 0270-9139, DOI: 10.1002/hep.28829

## Description

The present invention concerns methods for diagnosing liver fibrosis in a subject suffering from obesity, or for selecting a subject suffering from obesity for liver biopsy or for treatment.

Nonalcoholic fatty liver disease (NAFLD) is becoming the most prevalent liver disease in the world. Both excessive body mass index and visceral obesity are recognized risk factors for NAFLD. In patients with severe obesity undergoing bariatric surgery, the prevalence of NAFLD can exceed 90% and up to 5% of patients may have unsuspected cirrhosis. It is therefore very important to be able to detect these patients in order to monitor their liver function and to manage associated complications. Liver biopsy remains the gold standard for characterizing liver histology in patients with NAFLD. However, it is an invasive technique and subject to sampling errors and significant intra- and inter-observer variability. Hence, there is a major need for the development of non-invasive diagnostic strategies of liver fibrosis.

There has been intense interest in the development of non-invasive methods as an alternative to biopsy for the identification of advanced fibrosis in patients with NAFLD. These include the NAFLD Fibrosis Score, Enhanced Liver Fibrosis (ELF) panel and transient elastrography. The NAFLD Fibrosis Score is based on six readily available variables and has a ROC score of 0.85 for predicting advanced fibrosis. The ELF panel, which consists in the dosage of plasma levels of three matrix turnover proteins, has a ROC score of 0.90. Transient elastrography, which measures liver stiffness non-invasively, has been successful in identifying advanced fibrosis in NAFLD. However, it has a high failure rate in individuals with high BMI, and failure of liver stiffness measurement or unreliable results occur in 5% to 15% of patients of the general population, mainly due to obesity. Patent publications UA77798 and UA77726 disclose methods for non-invasive diagnostic of hepatic fibrosis in obese children and adolescents by quantitative analysis of the levels of fibronectin, collagen I or collagen IV in a blood sample.

There is thus still an important need for alternative non-invasive diagnosic methods of liver fibrosis, with high sensitivity and sensibility, in particular in obese patients.

The inventors have investigated here by quantitative (qPCR) and qualitative (16S targeted metagenomics sequencing) methods the relationship between blood microbiota, fecal microbiota and liver fibrosis in patients with severe obesity. Disease-specific alterations in blood microbiota could constitute a relevant, inexpensive and easy-to-sample approach for the diagnosis and characterization of liver fibrosis in this high risk population.

The present invention first arises from the unexpected finding by the inventors that obese patients with liver fibrosis have lower blood and feces bacterial diversity at all taxonomic levels and display differences in the proportions of particular bacterial taxa in the blood and feces. They showed that determination of bacterial diversity, typically through the determination of the Shannon index, and of proportions of particular bacterial taxa in biological samples of patients may thus be useful in the diagnosis of liver fibrosis in patients suffering from obesity.

The present disclosure thus relates to a method, in particular an *in vitro* method, for diagnosing liver fibrosis in a subject suffering from obesity, said method comprising the steps of:

a) determining bacterial diversity in a biological sample from the subject, and
b) based on the result of the bacterial diversity determination in step a), diagnosing liver fibrosis in the subject.

In a particular embodiment of the method of diagnosis step a) of determining bacterial diversity is performed by assessing the proportion of bacteria belonging to at least one particular bacterial taxon, with relation to the total bacterial level in said biological sample.

The inventors even showed that it is possible to obtain a method of diagnosis of liver fibrosis with very high sensitivity and specificity by combining the proportions of bacteria of a limited number of taxa in blood or feces.

The present disclosure thus also concerns a method, in particular an *in vitro* method, for diagnosing liver fibrosis in an obese subject, said method comprising the steps of:

a1) determining the proportion of bacteria belonging to a particular taxon and the proportion of bacteria belonging to at least one other particular taxon, in a biological sample of said subject,
a2) combining the proportions determined in step a1) to obtain a combination value, and
b) based on the combination value obtained in step a2), diagnosing liver fibrosis in the subject.

The present inventors particularly demonstrated that, among patients with high steatosis score, the presence of fibrosis correlates inversely with the blood level of *Variovorax* DNA. These results suggest that, among obese individuals, a high proportion of *Variovorax* bacteria in the blood protects against liver fibrosis.

Another aspect of the present disclosure thus concerns bacteria belonging to the *Variovorax* genera for use as a probiotic for preventing and/or treating liver fibrosis, in particular in an obese subject.

The inventors further demonstrated that these differences in bacterial diversity and in the proportions of particular bacterial taxa were associated with differences in the abundance of predicted bacterial gene functions from several metabolic pathways.

Using the PICRUSt (Phylogenetic Investigation of Communities by Reconstruction of Unobserved States) software to predict from the 16S metagenomic data the metagenome functional content (bacterial genes) present in the blood of obese patients suffering from liver fibrosis, they indeed showed that a number of predicted gene functions were significantly modified between groups of obese patients with or withour liver fibrosis. In particular, they showed that the groups of predicted bacterial gene functions involved in xenobiotics biodegradation and metabolism, metabolism of cofactors and vitamins and lipid metabolism are decreased in obese patients suffering from liver fibrosis. Inversely, they showed that the predicted relative abundance of bacterial gene functions of the Nif family, involved in nitrification, increased in obsese patients suffering from liver fibrosis.

The present disclosure thus also concerns a method, in particular an *in vitro* method, for diagnosing liver fibrosis in an obese subject, said method comprising the steps of:

a) determining the amount or relative proportion of bacterial gene functions from at least one metabolic pathway in a biological from the subject, and
b) based on the result of the bacterial gene functions determination in step a), diagnosing liver fibrosis in the subject.

The present disclosure finally pertains to a method for screening a probiotic, a prebiotic, a chemical compound or a biological compound suitable for preventing and/or treating liver fibrosis, comprising the steps of:

A) determining bacterial diversity in a biological sample from an obese subject suffering from liver fibrosis who has been treated with the candidate probiotic, prebiotic, chemical or biological compound, and
B) comparing said bacterial diversity with that of a control obese subject suffering from liver fibrosis who has not been treated with said candidate probiotic, prebiotic, chemical compound or biological compound, and
C) based on the result of the comparison at step B), determining if said candidate probiotic, prebiotic, chemical compound or biological compound is suitable for preventing and/or treating liver fibrosis.

In a particular embodiment of the method of screening step a) of determining bacterial diversity is performed by assessing the proportion of bacteria belonging to at least one particular bacterial taxon, with relation to the total bacterial level in said biological sample.

The present disclosure also concerns a method for screening a probiotic, a prebiotic, a chemical compound or a biological compound suitable for preventing and/or treating liver fibrosis, said method comprising the steps of:

A1) determining the proportion of bacteria belonging to a particular taxon and the proportion of bacteria belonging to at least one other particular taxon, in a biological sample from an obese subject suffering from liver fibrosis who has been treated with the candidate probiotic, prebiotic, chemical or biological compound,
A2) combining the proportions determined in step A1) to obtain a combination value,
B) comparing said combination value with that of a control obese subject suffering from liver fibrosis who has not been treated with said candidate probiotic, prebiotic, chemical compound or biological compound, and
C) based on the result of the comparison at step B), determining if said candidate probiotic, prebiotic, chemical compound or biological compound is suitable for preventing and/or treating liver fibrosis.

## Detailed description of the invention

*Fibrosis*

As used herein, the terms "fibrosis", "liver fibrosis" or "hepatic fibrosis" refer to a medical condition in which excessive connective tissue accumulates in the liver; this tissue represents scarring in response to chronic, repeated liver cell injury. Commonly, fibrosis progresses, disrupting hepatic architecture and eventually function, as regenerating hepatocytes attempt to replace and repair damaged tissue.

Liver fibrosis can be of any stage, in particular of stage 1 (perisinusoidal fibrosis), 2 (periportal fibrosis) or 3 (fibrosis in bridges).

Complications of liver fibrosis are well-known from the skilled person and include cirrhosis, liver failure, liver cancer, portal hypertension and associated complications (such as esophageal varices bleading or ascites) and hepatic encephalopathy.

*Subject*

In the context of the present invention, a "subject" denotes a human or non-human mammal, such as a rodent (rat, mouse, rabbit), a primate (chimpanzee), a feline (cat), or a canine (dog). Preferably, the subject is human. The subject according to the invention may be in particular a male or a female. Preferably, the subject is aged 40 to 60 years.

According to the present invention, the subject suffers from obesity (*i.e.* is obese).

As used herein, the term "obesity" refers to a medical condition in which excess body fat has accumulated to the extent that it may have an adverse effect on health, leading to reduced life expectancy and/or increased health problems. Obesity is typically determined by assessing the body mass index (BMI), a measurement which associates weight and height. In particular, people are defined as overweight if their BMI is between 25 kg/$m^2$ and 30 kg/$m^2$, and obese when it is greater than 30 kg/$m^2$.

In the context of the invention, the subject has preferably a body mass index (BMI) higher than 30 kg/$m^2$, more preferably than 37.5 kg/$m^2$, or even more preferably higher than 40 kg/$m^2$.

Preferably, the subject according to the invention suffers from non-alcoholic fatty liver disease (NAFL disease or NAFLD) at the time of sampling.

"Non-alcoholic fatty liver disease", "NAFL disease" or NAFLD is a term used herein to describe the accumulation of fat in the liver of people who drink little or no alcohol. In many cases, NAFL disease is linked to obesity. NAFL disease is common and, for most people, causes no signs and symptoms and no complications. But in some people with NAFL disease, the fat that accumulates can cause inflammation and scarring in the liver. This more serious form of NAFL disease is called non-alcoholic steatohepatitis (NASH).

In a particular embodiment, the subject has a body mass index (BMI) higher than 37.5 kg/$m^2$, in particular higher than 40 kg/$m^2$, and suffers from at least two metabolic co-morbidities selected from the group consisting of type 2 diabetes, hypertension and dyslipidemia.

As used herein, "diabetes" or "diabetes mellitus" denotes a metabolic disorder in which the pancreas produces insufficient amounts of insulin, or in which the cells of the body fail to respond appropriately to insulin thus preventing cells from absorbing glucose. As a result, glucose builds up in the blood. This high blood glucose level produces the classical symptoms of polyuria (frequent urination), polydipsia (increased thirst) and polyphagia (increased hunger).

Type 2 diabetes, also known as non-insulin-dependent diabetes mellitus (NIDDM) and adult-onset diabetes, is associated with predominant insulin resistance and thus relative insulin deficiency and/or a predominantly insulin secretory defect (or insulinopenia) with insulin resistance. More specifically, type 2 diabetes may be associated either with (i) a predominant insulin resistance with a moderate insulinopenia or with (ii) a moderate insulin resistance with a predominant insulinopenia.

As used herein, the term "hypertension" also referred to as "high blood pressure", "HTN" or "HPN", denotes a medical condition in which the blood pressure is chronically elevated. In the context of the invention, hypertension is preferably defined by systolic/diastolic blood pressure of at least 140/90 mmHg or being on antihypertensive medication.

As used herein, the term "dyslipidemia" denotes an elevation of plasma cholesterol, triglycerides or both, or a low high-density lipoprotein level that may contribute to the development of atherosclerosis.

In a particular embodiment, the subject is free of known systemic disease such as rheumatoid arthritis or systemic lupus erythematosus, serious chronic illness such as cardiovascular disease, in particular heart failure, cirrhosis, panhypopituitarism, autoimmune disease or cancer, and/or ethanol intake superior to 20 g per day.

In another particular embodiment, the subject according to the invention did not display infection symptom(s) during the month preceding sampling. Accordingly, the subject according to the invention preferably displays a plasma baseline C reactive protein concentration lower than 10 mg/l and/or does not present an abundant leukocyturia and/or does not take antiviral therapy.

As used herein, the term "C reactive protein" or "CRP" refers to a protein which is a member of the class of acute-phase reactants, as its levels rise dramatically during inflammatory processes occurring in the body. As known from the skilled person, CRP is typically a 224-residue protein with a monomer molar mass of 25 kDa, encoded by the *CRP* gene.

As used herein, the term "leukocyturia" refers to the presence of leukocytes in the urine of the subject. In particular, an abundant leukocyturia corresponds typically to the presence of more than 10 leukocytes/$mm^3$ in the urine.

*Biological sample*

As used herein, the term "biological sample" means a substance of biological origin. In particular, examples of biological samples include blood and components thereof and feces and its derivatives.

As used herein, the term "blood" refers either to blood or to any of its components such as serum, plasma, platelets, buffy coat (leucocytes), and erythrocytes. Also, the term "feces" refers either to feces or to any of its derivatives such as fecal water.

The biological sample according to the invention may be obtained from the subject by any appropriate means of sampling known from the skilled person.

*Bacterial diversity*

As used herein, the terms "bacterial diversity" refers to the level of bacterial taxa richness and optionally bacterial taxa abundance in a sample. As used herein, bacterial taxa richness refers to the number of bacterial taxa, in particular the total number of bacterial taxa, present in a sample.
By "taxon" or "taxa" is meant herein any taxonomic level, including phylum, class, order, family, genus or species of bacteria.
Bacterial diversity may be determined at any taxonomic level. In particular, bacterial diversity may be determined at the phylum level, at the class level, at the order level, at the family level, at the genus level, at the species level or at the operational taxonomic unit (OTU) level. Preferably, bacterial diversity is determined at the phylum level, at the class level, at the family level, at the genus level, at the species level or at the OTU level. In a particular embodiment, bacterial diversity is determined at the phylum level. In another particular embodiment, bacterial diversity is determined at the family level. In another particular embodiment, bacterial diversity is determined at the genus or at the species level. In another particular embodiment, bacterial diversity is determined at the class level or at the OTU level. In a particularly preferred embodiment, bacterial diversity is determined at the class level.
Techniques to determine bacterial diversity are well-known from the skilled person and include the determination of species richness indices such as OTU richness estimators, the Margalef index, the Q statistic or the Shannon index, or of evenness and dominance indices such as the Shannon evenness index, the Lloyd and Ghelardi index, the Simpson's index or the Berger-Parker index.
Preferably, the bacterial diversity is determined by determining the Shannon index.
As well-known from the skilled person, the Shannon index or H' (also called Shannon's diversity index, Shannon-Wiener index, Shannon-Weaver index, or Shannon entropy) is calculated as follows:

$$H' = -\sum_{i=1}^{R} p_i \ln p_i$$

where

$p_i$ is the proportion of bacteria belonging to the $i^{th}$ taxon (estimated using $n_i/N$ where $n_i$ is the number or concentration of bacteria in a taxon and N is the total number or concentration of bacteria), and
R is the number of taxa in the sample.

In particular embodiments of the methods disclosed herein. the steps of determining bacterial diversity are performed by assessing the proportion of bacteria belonging to at least one particular bacterial taxon, with relation to the total bacterial level in said biological sample.
The proportion of bacteria belonging to a particular taxon with relation to the total bacterial level may be determined by measuring the level (quantity or concentration) of all the bacteria belonging to said particular taxon or taxa in the biological sample, measuring the total level (quantity or concentration) of bacteria present or detected in said biological sample, and dividing the level of the bacteria belonging to said particular taxon or taxa by the total level of bacteria in the sample. This proportion may optionally be expressed as a percentage of the total level of bacteria present in said biological sample.
In the context of the invention, determining the proportion of the bacteria belonging to a particular taxon thus means measuring the level of bacteria belonging to this entire taxon (i.e. belonging to all the sub-taxa included in this taxon) in the biological sample, and dividing this level by the total level of bacteria in the sample.
The level of bacteria (i.e. either the level of bacteria belonging to a particular taxon, or the total level of bacteria in a sample) may be determined by any method enabling the detection and quantification of bacteria. Preferably, the level of bacteria may be determined by PCR, by metagenomic sequencing, an antibody-based method such as e.g. an ELISA, by DGGE (Denaturing Gradient Gel Electrophoresis) or by TRFLP (Terminal Restriction Fragment Length Polymorphism).
Preferably, the level of bacteria (i.e. either the level of bacteria belonging to a particular taxon, or the total level of bacteria in a sample) is measured by polymerase chain reaction (PCR), more preferably by real-time PCR (qPCR, Real-Time RT-PCR or RT-qPCR).
In particular, the total level of bacteria in a sample is preferably measured by real-time PCR.
As used herein, "real-time PCR", "real-time quantitative PCR", "real-time polymerase chain reaction" or "kinetic polymerase chain reaction" refers to a laboratory technique based on the polymerase chain reaction, which is used to amplify and simultaneously quantify a targeted DNA molecule. It enables both detection and quantification (as absolute number of copies or relative amount when normalized to DNA input or additional normalizing genes) of a specific sequence in a sample. Two common methods of quantification are the use of fluorescent dyes that intercalate with doublestranded DNA, and modified DNA oligonucleotide probes that emit fluorescence when hybridized with a complementary DNA.

In the context of the invention, the determination of the levels of bacteria may be performed by detecting and amplifying, in particular by real-time PCR, any bacterial nucleic acid sequence from said bacteria. Said bacterial nucleic acid sequence may be for instance the nucleic acid encoding the bacterial 16S ribosomal RNA.

As used herein, the expression "nucleic acid encoding the bacterial 16S ribosomal RNA" or "16S rDNA" refers to the gene encoding the 16S ribosomal RNA constituted of about 1500 nucleotides, which is the main component of the small prokaryotic ribosomal subunit (30S). 16S rDNA is highly conserved among bacteria. The reference *Escherichia coli* 16S rDNA gene sequence corresponds to SEQ ID NO: 1 (called *rrs*). In the context of the invention, 16S rDNA refers to any sequence corresponding to SEQ ID NO: 1 in other bacterial strains.

In the context of the invention, the determination of the levels of bacteria may be performed by detecting and amplifying, in particular by real-time PCR, bacterial 16S rDNA, using primers hybridizing, under high stringency conditions, with the bacterial 16S rDNA sequence from bacteria of almost any taxon, or alternatively using primers hybridizing, under high stringency conditions, with the bacterial 16S rDNA sequence from bacteria of specific taxa.

Preferably, the level of bacterial 16S rDNA may for instance be the concentration of bacterial 16S rDNA expressed by the number of copies per μl, ml, μg or mg of biological sample of the subject. Also preferably, the level of bacterial 16S rDNA may be the ratio of the quantity of bacterial 16S rDNA to the quantity of total DNA (16S rDNA / total DNA ratio), expressed for instance by the number of copies per μg or mg of total DNA in the biological sample of the subject.

In the context of the invention, when the 16S rDNA bacterial sequence is detected by real-time PCR, in particular for determining the total level of bacteria in a sample, the real-time PCR is preferably performed using forward and reverse primers targeting the V3-V4 hyper-variable regions of the 16S rDNA, more preferably using the forward and reverse primers EUBF of sequence 5'-TCCTACGGGAGGCAGCAGT-3' (SEQ ID NO: 2) and EUBR of sequence 5'-GGACTACCAGGGTATCTAATCCTGTT-3' (SEQ ID NO: 3). Typically, the amplification is performed using 5 ng of total of DNA in a reaction volume of 12.5 μl, using for instance Sybr Green RT-qPCR technologies, typically with the following cycle: hold stage of 5 min at 95°C, then 40 cycles of 15 sec at 95°C, 1 min at 63°C and 1 min at 72°C.

Also preferably, the levels of bacteria, in particular in a blood sample, may be determined by metagenomic sequencing (such as using the MiSeq® sequencing system). In a preferred embodiment, the levels of bacteria are determined by metagenomics sequencing targeting 16S rDNA, as defined above. In the context of the invention, when the 16S rDNA bacterial sequence is detected by metagenomic sequencing, in particular in a blood sample of the patient, the V3-V4 hyper-variable regions of the 16S rDNA may typically be amplified for MiSeq sequencing, preferably using a two-step PCR strategy. The first PCR may typically be performed using primers which include the first part of the MiSeq P5/P7 adaptors, such as the Vaiomer 1F primer of sequence CTTTCCCTACACGACGCTCTTCCGATCTTCCTACGGGAGGCAGCAGT (SEQ ID NO: 4) and the Vaiomer 1R primer of sequence GGAGTTCAGACGTGTGCTCTTCCGATCTGGACTACCAGGGTATCTAATCCTGTT (SEQ ID NO: 5). As well-known from the skilled person, the MiSeq P5/P7 adaptors are parts of primers which enable generating a sequence which fixes amplified DNA to the sequencer. A first part of the adaptors is typically present in the first set of primers and the second part of the adaptors is typically present in the second set of primers. Sample multiplexing may typically be performed using indices, in particular tailor-made 6 bases unique indices, which may be added during the second PCR step, preferably at the same time as the second part of the P5/P7 adaptors with primers which preferably hybridize with a part of the sequence generated by the first set of primers, for instance with the primers Vaiomer 2F of sequence AATGATACGGCGACCACCGAGATCTACACTCTTTCCCTACACGAC (SEQ ID NO: 6) and Vaiomer 2R of sequence CAAGCAGAAGACGGCATACGAGAT (SEQ ID NO: 7)-index-GTGACTGGAGTTCAGACGTGT (SEQ ID NO: 8). Both PCR reactions may typically be carried out on a Veriti Thermal Cycler (LifeTechnologies) preferably followed by an amplicons purification for example using the magnetic beads Agencourt AMPure XP - PCR Purification (Beckman Coulter, CA, USA). The 16S rDNA amplicons are then typically sequenced preferably using the Illumina MiSeq system and preferably Illumina MiSeq Reagent Kit v3 (2x300 bp Paired-End Reads, 15 Gb output, Illumina, CA, USA).

Alternatively, the levels of bacteria, in particular in blood or feces sample, may be determined by different techniques of metagenomics analysis (whole genome sequencing, 16S targeted metagenomic sequencing, metabarcoding, 16S pyrosequencing) using different sequencing system such as, but not limited, to Illumina Miseq , Illumina Hiseq, Roche 454, Thermo Fischer scientific Ion Torrent, Pacific Bioscience Pacbio, Oxford Nanopore Technologies MinION/Promethion. Alternatively, the levels of bacteria in blood or feces sample may be determined by Phylochip microarray, DNA chip or RNA chip. In a preferred embodiment, the levels of bacteria are determined by metagenomics sequencing targeting 16S rDNA, as defined above, and pyrosequencing, or by using the MiSeq® sequencing system. In the context of the invention, when the 16S rDNA bacterial sequence is detected by 16S metagenomic sequencing with pyrosequencing, in particular in a feces sample of the patient, the whole 16S bacterial DNA V1-V3 region may typically be targeted by the primers 28F of sequence GAGTTTGATCNTGGCTCAG (SEQ ID NO: 9) and 519R of sequence GTNTTACNGCGGCKGCTG (SEQ ID NO: 10) and preferably sequenced by pyrosequencing.

Alternatively, the levels of bacteria belonging to a particular bacterial taxon may be determined by detecting and amplifying bacterial nucleic acid sequences specific of said taxon. Said bacterial nucleic acid sequences specific of said taxon may be for instance a particular region of the nucleic acid encoding the bacterial 16S ribosomal RNA.

*Bacterial taxa*

As detailed above, the bacterial diversity may be determined at any taxonomic level.

In a particular embodiment, the bacterial diversity is determined at the phylum level.

The present inventors demonstrated that the blood of obese subjects comprises bacterial DNA from bacteria belonging to the *Actinobacteria* phylum, the *Bacteroidetes* phylum, the *Firmicutes* phylum and the *Proteobacteria* phylum, while the feces of obsese subjects further comprises bacterial DNA from bacteria belonging to the *Fusobacteria* phylum and the *Verrucomicrobia* phylum.

Accordingly, in a particular embodiment, the method of diagnosis disclosed herein comprises the steps of:

a) determining the proportion of bacteria belonging to at least one bacterial taxon included in the *Actinobacteria* phylum, the *Bacteroidetes* phylum, the *Firmicutes* phylum or the *Proteobacteria* phylum, or optionally in the *Fusobacteria* phylum or the *Verrucomicrobia* phylum, in relation to the total bacterial level, in a biological sample from the subject, and
b) based on the result of the measurement in step a), diagnosing liver fibrosis in said subject.

In a particular embodiment, the method of diagnosis disclosed herein comprises the steps of:

a) determining the proportion of bacteria belonging to at least one bacterial taxon included in the *Actinobacteria* phylum, the *Bacteroidetes* phylum, the *Firmicutes* phylum or the *Proteobacteria* phylum, with relation to the total bacterial level, in a blood sample of the subject, or
determining the proportion of bacteria belonging to at least one bacterial taxon included in the *Actinobacteria* phylum, the *Bacteroidetes* phylum, the *Firmicutes* phylum, the *Proteobacteria* phylum, the *Fusobacteria* phylum or the *Verrucomicrobia* phylum, with relation to the total bacterial level, in a feces sample of the subject; and
b) based on the result of the measurement in step a), diagnosing liver fibrosis in said subject.

In another embodiment, the bacterial diversity is determined at the class level.

The inventors demonstrated that obese subjects with liver fibrosis displayed a significantly different proportion of bacteria belonging to the *Clostridia* class and to the *Erysipelotrichia* class in their feces.

Accordingly, in a particular embodiment, the method of diagnosis disclosed herein comprises the steps of:

a) determining the proportion of bacteria belonging to at least one bacterial taxon included in the *Clostridia* class or in the *Erysipelotrichia* class, in relation to the total bacterial level, in a biological sample, in particular in a feces sample, from the subject, and
b) based on the result of the measurement in step a), diagnosing liver fibrosis in said subject.

In another embodiment, the bacterial diversity is determined at the order level.

The inventors demonstrated that obese subjects with liver fibrosis displayed a significantly different proportion of bacteria belonging to the *Actinomycetales* order or to the *Sphingomonadales* order in their blood, and a significantly different proportion of bacteria belonging to the *Erysipelotrichales* order, to the *Bacteroidales* order, to the *Coriobacteriales* order or to the *Clostridiales* order in their feces.

Accordingly, in a particular embodiment, the method of diagnosis disclosed herein comprises the steps of:

a) determining the proportion of bacteria belonging to at least one bacterial taxon included in the *Actinomycetales* order, the *Sphingomonadales* order, the *Erysipelotrichales* order, the *Bacteroidales* order, the *Coriobacteriales* order or the *Clostridiales* order, in relation to the total bacterial level, in a biological sample from the subject, and
b) based on the result of the measurement in step a), diagnosing liver fibrosis in said subject.

In a particular embodiment, the method of diagnosis disclosed herein comprises the step of:

a) determining the proportion of bacteria belonging to at least one bacterial taxon included in the *Actinomycetales* order or the *Sphingomonadales* order, with relation to the total bacterial level, in a blood sample of the subject, or
determining the proportion of bacteria belonging to at least one bacterial taxon included in the *Erysipelotrichales* order, the *Bacteroidales* order, the *Coriobacteriales* order or the *Clostridiales* order, with relation to the total bacterial level, in a feces sample of the subject; and
b) based on the result of the measurement in step a), diagnosing liver fibrosis in said subject.

In another embodiment, the bacterial diversity is determined at the family level.
The inventors demonstrated that obese subjects with liver fibrosis displayed a significantly different proportion of bacteria belonging to the *Bradyrhizobiaceae* family or to the *Sphingomonadaceae* family in their blood, and a significantly different proportion of bacteria belonging to the *Fusobacteriaceae* family, to the *Coriobacteriaceae* family, to the *Ruminococcaceae* family or to the *Lachnospiraceae* family in their feces.
Accordingly, in a particular embodiment, the method of diagnosis disclosed herein comprises the steps of:

a) determining the proportion of bacteria belonging to at least one bacterial taxon included in the *Bradyrhizobiaceae* family, the *Sphingomonadaceae* family, the *Fusobacteriaceae* family, the *Coriobacteriaceae* family, the *Ruminococcaceae* family or the *Lachnospiraceae* family in relation to the total bacterial level, in a biological sample from the subject, and
b) based on the result of the measurement in step a), diagnosing liver fibrosis in said subject.

In a particular embodiment, the method of diagnosis disclosed herein comprises the step of:

a) determining the proportion of bacteria belonging to at least one bacterial taxon included in the *Bradyrhizobiaceae* family or in the *Sphingomonadaceae* family, with relation to the total bacterial level, in a blood sample of the subject, or determining the proportion of bacteria belonging to at least one bacterial taxon included in the *Fusobacteriaceae* family, the *Coriobacteriaceae* family, the *Ruminococcaceae* family or the *Lachnospiraceae* family, with relation to the total bacterial level, in a feces sample of the subject; and
b) based on the result of the measurement in step a), diagnosing liver fibrosis in said subject.

In another embodiment, the bacterial diversity is determined at the genera level.
The inventors demonstrated that obese subjects with liver fibrosis displayed a significantly different proportion of bacteria belonging to the *Sphingomonas* genera or to the *Bosea* genera in their blood, and a significantly different proportion of bacteria belonging to the *Dorea* genera in their feces.
Accordingly, in a particular embodiment, the method of diagnosis disclosed herein comprises the steps of:

a) determining the proportion of bacteria belonging to at least one bacterial taxon included in the *Sphingomonas* genera, the *Bosea* genera or in the *Dorea* genera, in relation to the total bacterial level, in a biological sample from the subject, and
b) based on the result of the measurement in step a), diagnosing liver fibrosis in said subject.

In a particular embodiment, the method of diagnosis disclosed herein comprises the step of:

a) determining the proportion of bacteria belonging to at least one bacterial taxon included in the the *Sphingomonas* genera or in the the *Bosea* genera, with relation to the total bacterial level, in a blood sample of the subject, or determining the proportion of bacteria belonging to at least one bacterial taxon included in the *Dorea* genera, with relation to the total bacterial level, in a feces sample of the subject; and
b) based on the result of the measurement in step a), diagnosing liver fibrosis in said subject.

In another embodiment, the bacterial diversity is determined at the species level.
The inventors demonstrated that obese subjects with liver fibrosis displayed a significantly different proportion of bacteria belonging to the *Variovorax paradoxus* species in their blood.
Accordingly, in a particular embodiment, the method of diagnosis disclosed herein comprises the steps of:

a) determining the proportion of bacteria belonging to at least the *Variovorax paradoxus* species, in relation to the total bacterial level, in a biological sample, in particular a blood sample, from the subject, and
b) based on the result of the measurement in step a), diagnosing liver fibrosis in said subject.

In other embodiments, the bacterial diversity is determined at different taxonomic levels. For example, the bacterial diversity may be determined by determining the proportions of particular phyla and particular genera, or the proportions of particular phyla and particular species.

*Combinations*

As mentioned above, the inventors showed that it is possible to obtain a method of diagnosis of liver fibrosis with very high sensitivity and sensibility by combining the proportions of bacteria present in blood or feces of a limited number of taxa.

By "combining" or "combination" is meant herein associating the proportions of bacteria belonging to specific taxa and/or the total level of bacteria into algorithms or models, such as regression models, generating combination values. Preferably, said combination is designed to obtain a combination value that gives a negative predictive value (NPV) and a positive predictive value (PPV) superior to 80%, preferably superior to 85%, more preferably superior to 90%, even more preferably superior to 95% in the targeted population. As used herein, the term "targeted population" refers to a population constituted of subjects who share certain biological parameters such as e.g. gender, age group, or certain environmental parameters such as e.g. geographical region.

By "combination value" is meant herein the value obtained using the algorithm or model associating said proportions of bacteria and/or total levels of bacteria.

It is possible to combine the proportions of bacteria belonging to taxa of any level, including of taxa of different levels, such as combining the proportions of bacteria belonging to particular phyla, particular classes, particular orders, particular families, particular genera or particular species, or combining the proportion of bacteria belonging to a particular genera with the proportion of bacteria belonging to another particular genera, or combining the proportion of bacteria belonging to a particular genera with the proportion of bacteria belonging to a particular phylum.

The inventors demonstrated that high sensitivity and specificity could be obtained by combining the proportion of the *Variovorax* genera and the proportion of bacteria belonging to at least one other particular taxon.

Accordingly, in the methods disclosed herein involving a combination step, step a1) preferably comprises determining the proportion of *Variovorax* genera and the proportion of bacteria belonging to at least one other particular taxon, with relation to the total bacterial level, in a biological sample of said subject.

In other preferred embodiments of the methods disclosed herein step a1) preferably comprises determining the proportion of *Variovorax* genera, and the proportion of bacteria belonging to at least one other taxon selected from the group consisting of *Sphingomonas* genera, *Stenotrophomonas* genera, *Bosea* genera, *Micrococcus* genera, *Proteobacteria* phylum and *Actinobacteria* phylum, with relation to the total bacterial level, in a biological sample of said subject.

In particularly preferred embodiments of the methods disclosed herein, step a1) preferably comprises determining the proportion of *Variovorax* genera, and the proportion of bacteria belonging to at least one, two, three or four taxa selected from the group consisting of *Sphingomonas* genera, *Stenotrophomonas* genera, *Bosea* genera and *Micrococcus* genera, with relation to the total bacterial level, in a biological sample of said subject.

In still particularly preferred embodiments of the methods of the invention, step a1) preferably comprises determining the proportion of *Variovorax* genera, the proportion of bacteria belonging to the *Sphingomonas* genera, the proportion of bacteria belonging to the *Stenotrophomonas* genera, the proportion of bacteria belonging to the *Bosea* genera and the proportion of bacteria belonging to the *Micrococcus* genera, with relation to the total bacterial level, in a biological sample of said subject.

In other particularly preferred embodiments of the methods disclosed herein, step a1) preferably comprises determining the proportion of *Variovorax* genera, and the proportion of bacteria belonging to at least one or two taxa selected from the group consisting of the *Proteobacteria* phylum and *Actinobacteria* phylum, with relation to the total bacterial level, in a biological sample of said subject.

In still particularly preferred embodiments of the methods of the invention, step a1) preferably comprises determining the proportion of *Variovorax* genera, the proportion of bacteria belonging to the *Proteobacteria* phylum and the proportion of bacteria belonging to the *Actinobacteria* phylum, with relation to the total bacterial level, in a biological sample of said subject.

The inventors further demonstrated that a still higher specificity and sensitivity could be obtained by further combining, with these proportions, the total bacterial level in the biological sample.

Accordingly, in particularly preferred embodiments of the methods of the invention, step a1) further comprises determining the total bacterial level, in particular using methods as defined in the section *"Bacterial diversity"* above, in the biological sample of said subject, and step a2) comprises combining the proportions and the total bacterial level determined in step a1).

In particularly preferred embodiments of the methods disclosed herein, step a1) thus comprises determining the proportion of *Variovorax* genera, the proportion of bacteria belonging to at least one other taxon selected from the group consisting of *Sphingomonas* genera, *Stenotrophomonas* genera, *Bosea* genera, *Micrococcus* genera, *Proteobacteria* phylum and *Actinobacteria* phylum, with relation to the total bacterial level, and the total bacterial level in a biological sample of said subject, and step a2) comprises combining the proportions and the total bacterial level determined in step a1).

In still particularly preferred embodiments of the methods disclosed herein, step a1) thus comprises determining the proportion of *Variovorax* genera, the proportion of bacteria belonging to at least one, two, three or four taxa selected from the group consisting of *Sphingomonas* genera, *Stenotrophomonas* genera, *Bosea* genera and *Micrococcus* genera, with relation to the total bacterial level, and the total bacterial level in a biological sample of said subject, and step a2) comprises combining the proportions and the total bacterial level determined in step a1).

In still particularly preferred embodiments of the methods of the invention, step a1) comprises determining the proportion of *Variovorax* genera, the proportion of bacteria belonging to the *Sphingomonas* genera, the proportion of bacteria belonging to the *Stenotrophomonas* genera, the proportion of bacteria belonging to the *Bosea* genera, the proportion of bacteria belonging to the *Micrococcus* genera, with relation to the total bacterial level, and the total bacterial level in a biological sample of said subject, and step a2) comprises combining the proportions and the total bacterial level determined in step a1).

In still particularly preferred embodiments of the methods disclosed herein, step a1) comprises determining the proportion of *Variovorax* genera, the proportion of bacteria belonging to at least one or two taxa selected from the group consisting of the *Proteobacteria* phylum and *Actinobacteria* phylum, with relation to the total bacterial level, and the total bacterial level in a biological sample of said subject, and step a2) comprises combining the proportions and the total bacterial level determined in step a1).

In most preferred embodiments of the methods of the invention, step a1) comprises determining the proportion of *Variovorax* genera, the proportion of bacteria belonging to the *Proteobacteria* phylum, the proportion of bacteria belonging to the *Actinobacteria* phylum, with relation to the total bacterial level, and the total bacterial level in a biological sample of said subject, and step a2) comprises combining the proportions and the total bacterial level determined in step a1).

*Bacterial gene functions*

As detailed above, the inventors demonstrated that a number of predicted bacterial gene functions from metabolic pathways were significantly modified between groups of obese patients with or without liver fibrosis.

By "bacterial gene function" is meant herein any bacterial gene or groups of bacterial genes implicated in a specific bacterial function or specific metabolic pathway.

Said bacterial gene function may be measured directly (for example by qPCR or whole genome sequencing) or predicted for example based on the assigned taxa found by 16S targeted metagenomic sequencing.

Bacterial gene functions may be gathered according to the metabolic pathways in which they are involved. Examples of metabolic pathways are well-known from the skilled person and include xenobiotics biodegradation and metabolism, metabolism of cofactors and vitamins, lipid metabolism and nitrification (through the Nif family). In a particular embodiment, said at least one metabolic pathway is selected from the group consisting of xenobiotics biodegradation and metabolism, metabolism of cofactors and vitamins and lipid metabolism. In a particularly preferred embodiment, said at least one metabolic pathway is the xenobiotics biodegradation and metabolism pathway.

The amount or relative proportions of bacterial gene functions may be determined by any suitable method such as by whole genome sequence sequencing, quantitative PCR or using sequenced based function and metabolic inference softwares such as for example PICRUST (disclosed in Langille et al. (2013) Nat. Biotechnol. 31:814-821), Tax4Fun (described in Aβhauer et al. (2015) Bioinformatics 31:2882-2884) or PAPRICA (described in Bowman et al. (2015) PLoS ONE 10:e0135868).

In a particular embodiment, the amount of bacterial gene functions is determined using the PICRUSt software.

*Methods*

The present disclosure concerns a method for diagnosing liver fibrosis, as defined in the section *"Fibrosis"* above, in an obese subject, as defined in the section *"Subject"* above, said method comprising the steps of:

a) determining bacterial diversity, as defined in the section *"Bacterial diversity"* above, in a biological sample, as defined in the section *"Biological sample"* above, from the subject, and
b) based on the result of the bacterial diversity determination in step a), diagnosing liver fibrosis in the subject.

The present disclosure also concerns a method for diagnosing liver fibrosis, as defined in the section *"Fibrosis"* above, in an obese subject, as defined in the section *"Subject"* above, said method comprising the steps of:

a1) determining the proportion of bacteria belonging to a particular taxon and the proportion of bacteria belonging to at least one other particular taxon, as defined in the section *"Bacterial diversity"* above in a biological sample, as defined in the section *"Biological sample"* above of said subject,

a2) combining the proportions determined in step a1), as defined in the section *"Combination"* above, to obtain a combination value, and
b) based on the combination value obtained in step a2), diagnosing liver fibrosis in the subject.

The present disclosure further concerns a method for diagnosing liver fibrosis, as defined in the section *"Fibrosis"* above, in an obese subject, as defined in the section *"Subject"* above, said method comprising the steps of:

a) determining the amount or relative proportion of bacterial gene functions from at least one metabolic pathway, as defined in the section *"Bacterial gene functions"* above, in a biological sample, as defined in the section *"Biological sample"* above from said subject, and
b) based on the result of the bacterial gene functions determination in step a), diagnosing liver fibrosis in the subject.

Liver biopsy is the standard for diagnosing liver fibrosis and for diagnosing the underlying liver disorder causing fibrosis. However, liver biopsy is invasive. Therefore, liver biopsy should not be done systematically, but rather to confirm the presence of liver fibrosis in a subject.
The present disclosure thus also concerns an *in vitro* method for selecting an obese subject, as defined in the section *"Subject"* above, for liver biopsy, said method comprising the steps of:

a) determining bacterial diversity, as defined in the section *"Bacterial diversity"* above, in a biological sample, as defined in the section *"Biological sample"* above, from the subject, and
b) based on the result of the bacteria diversity determination in step a), selecting the subject suffering from obesity to undergo liver biopsy.

In a particular embodiment of the method of selection disclosed herein, step a) of determining bacterial diversity is performed by assessing the proportion of bacteria belonging to at least one particular bacterial taxon, as defined in the section *"Bacterial diversity"* above, with relation to the total bacterial level in said biological sample.
The present disclosure also concerns a method, for selecting an obese subject, as defined in the section *"Subject"* above, for liver biopsy, said method comprising the steps of:

a1) determining the proportion of bacteria belonging to a particular taxon and the proportion of bacteria belonging to at least one other particular taxon, as defined in the section *"Bacterial diversity"* above, in a biological sample, as defined in the section *"Biological sample"* above, of said subject,
a2) combining the proportions determined in step a1), as defined in the section *"Combination"* above, to obtain a combination value, and
b) based on the combination value determined in step a2), selecting the subject to undergo liver biopsy.

The present disclosure also concerns an *in vitro* method for selecting an obese subject, as defined in the section *"Subject"* above, for liver biopsy, said method comprising the steps of:

a) determining the amount or relative proportion of bacterial gene functions from at least one metabolic pathway, as defined in the section *"Bacterial gene functions"* above, in a biological sample, as defined in the section *"Biological sample"* above from said subject, and
b) based on the result of the bacterial gene functions determination in step a), selecting the subject suffering from obesity to undergo liver biopsy.

The inventors further demonstrated that bacterial diversity in blood or feces correlated with the seriousness of liver fibrosis. Using the method of the invention, it is thus possible to select an obese subject for treatment regimen targeting liver fibrosis and/or its complications.
The present disclosure thus also concerns an *in vitro* method for selecting an obese subject, as defined in the section *"Subject"* above, for treatment regimen targeting liver fibrosis and/or its complications, said method comprising the steps of:

a) determining bacterial diversity, as defined in the section *"Bacterial diversity"* above, in a biological sample, as defined in the section *"Biological sample"* above, from the subject, and
b) based on the result of the bacterial diversity determination in step a), selecting the subject suffering from obesity to undergo treatment regimen targeting liver fibrosis and/or its complications.

In a particular embodiment of the method of selection disclosed herein, step a) of determining bacterial diversity is performed by assessing the proportion of bacteria belonging to at least one particular bacterial taxon, as defined in the section *"Bacterial diversity"* above, with relation to the total bacterial level in said biological sample.

The present disclosure also concerns a method for selecting an obese subject, as defined in the section *"Subject"* above, for treatment regimen targeting liver fibrosis and/or its complications, said method comprising the steps of:

a1) determining the proportion of bacteria belonging to a particular taxon and the proportion of bacteria belonging to at least one other particular taxon, as defined in the section *"Bacterial diversity"* above, in a biological sample, as defined in the section *"Biological sample"* above, of said subject,
a2) combining the proportions determined in step a1), as defined in the section *"Combination"* above, to obtain a combination value, and
b) based on the combination value determined in step a2), selecting the subject to undergo treatment regimen targeting liver fibrosis and/or its complications.

The present disclosure also concerns an *in vitro* method for selecting an obese subject, as defined in the section *"Subject"* above, for treatment regimen targeting liver fibrosis and/or its complications, said method comprising the steps of:

a) determining the amount or relative proportion of bacterial gene functions from at least one metabolic pathway, as defined in the section *"Bacterial gene functions"* above, in a biological sample, as defined in the section *"Biological sample"* above from said subject, and
b) based on the result of the bacterial gene functions determination in step a), selecting the subject suffering from obesity to undergo treatment regimen targeting liver fibrosis and/or its complications.

In particular embodiments, the methods of diagnosing liver fibrosis in an obese subject as defined above, or the method for selecting a subject suffering from obesity for treatment regimen targeting liver fibrosis and/or its complications as defined above, further comprise a step c) of submitting the subject to a treatment regimen targeting liver fibrosis and/or its complications, if liver fibrosis has been diagnosed in step b).

The disclosure also relates to an *in vitro* method for determining if an obese patient, as defined in the section *"Subject"* above, suffering from liver fibrosis and/or its complications, responds to a drug treatment targeting liver fibrosis and/or its complications, said method comprising the steps of:

a) determining bacterial diversity, as defined in the section *"Bacterial diversity"* above, in a biological sample as defined in the section *"Biological sample"* above, from the subject before drug treatment,
a') determining bacterial diversity, as defined in the section *"Bacterial diversity"* above, in a biological sample, as defined in the section *"Biological sample"* above, from the subject after drug treatment, and
b) based on the result of the bacterial diversity determinations in steps a) and a'), determining if the patient responds to said drug treatment.

In a particular embodiment of the method of treatment reponsiveness disclosed herein, steps a) and a') of determining bacterial diversities are performed by assessing the proportion of bacteria belonging to at least one particular bacterial taxon, as defined in the section *"Bacterial diversity"* above, with relation to the total bacterial level in said respective biological samples.

The present disclosure also concerns an *in vitro* method for determining if an obese patient, as defined in the section *"Subject"* above, suffering from liver fibrosis and/or its complications responds to a drug treatment targeting liver fibrosis and/or its complications, said method comprising the steps of:

a1) determining the proportion of bacteria belonging to a particular taxon and the proportion of bacteria belonging to at least one other particular taxon, as defined in the section *"Bacterial diversity"* above, in a biological sample, as defined in the section *"Biological sample"* above, from the subject before drug treatment,
a2) combining the proportions determined in step a1), as defined in the section *"Combination"* above, to obtain a combination value before treatment,
a'1) determining the proportion of bacteria belonging to a particular taxon and the proportion of bacteria belonging to at least one other particular taxon, as defined in the section *"Bacterial diversity"* above, in a biological sample, as defined in the section *"Biological sample"* above, from the subject after drug treatment,
a'2) combining the proportions determined in step a'1), as defined in the section *"Combination"* above, to obtain a combination value after treatment,
b) based on the combination values determined in steps a2) and a'2), determining if the patient responds to said drug treatment.

The present disclosure also concerns an *in vitro* method for determining if an obese patient, as defined in the section *"Subject"* above, suffering from liver fibrosis and/or its complications responds to a drug treatment targeting liver fibrosis and/or its complications, said method comprising the steps of:

a) determining the amount or relative proportion of bacterial gene functions from at least one metabolic pathway, as defined in the section *"Bacterial gene functions"* above, in a biological sample, as defined in the section *"Biological sample"* above, from the subject before drug treatment,
a') the amount or relative proportion of bacterial gene functions from at least one metabolic pathway, as defined in the section *"Bacterial gene functions"* above, in a biological sample, as defined in the section *"Biological sample"* above, from the subject after drug treatment, and
b) based on the result of the bacterial gene functions determination in steps a) and a'), determining if the patient responds to said drug treatment.

If the patient is determined as not responding to said drug treatment at step b), it is possible to adapt the treatment of said patient targeting liver fibrosis and/or its complications.
Preferably, "adapting the treatment targeting liver fibrosis and/or its complications" means changing the drug used to treat the patient, or increasing or reducing the dose, the administration frequency, or changing the administration route of the drug treatment. It may also mean submitting said subject to clinical care including for instance ultrasounds for detection of liver cancer, or gastric fibroscopy for detection of varices of the esophagus. Such clinical care is well-known for the skilled person.
The present disclosure also concerns a method for treating an obese subject, as defined in the section *"Subject"* above, suffering from liver fibrosis and/or its complications, said method comprising the steps of:

a) determining bacterial diversity, as defined in the section *"Bacterial diversity"* above, in a biological sample, as defined in the section *"Biological sample"* above, from the subject,
b) based on the result of the bacterial diversity determination in step a), selecting the subject to undergo drug treatment targeting liver fibrosis and/or its complications, and
c) administering to the subject selected in step b) a drug treatment targeting liver fibrosis and/or its complications.

In a particular embodiment of the method of treatment disclosed herein, step a) of determining bacterial diversity is performed by assessing the proportion of bacteria belonging to at least one particular bacterial taxon, as defined in the section *"Bacterial diversity"* above, with relation to the total bacterial level in said biological sample.
The present disclosure also concerns a method for treating an obese subject, as defined in the section *"Subject"* above, suffering from liver fibrosis and/or its complications, said method comprising the steps of:

a1) determining the proportion of bacteria belonging to a particular taxon and the proportion of bacteria belonging to at least one other particular taxon, as defined in the section *"Bacterial diversity"* above, in a biological sample, as defined in the section *"Biological sample"* above, of said subject,
a2) combining the proportions determined in step a1), as defined in the section *"Combinations"* above, to obtain a combination value, and
b) based on the combination value determined in step a2), selecting the subject to undergo drug treatment targeting liver fibrosis and/or its complications, and
c) administering to the subject selected in step b) a drug treatment targeting liver fibrosis and/or its complications.

The present disclosure also concerns a method for treating an obese subject, as defined in the section *"Subject"* above, suffering from liver fibrosis and/or its complications, said method comprising the steps of:

a) determining the amount or relative proportion of bacterial gene functions from at least one metabolic pathway, as defined in the section *"Bacterial gene functions"* above, in a biological sample, as defined in the section *"Biological sample"* above from said subject, and
b) based on the result of the bacterial gene functions determination in step a), selecting the subject to undergo drug treatment targeting liver fibrosis and/or its complications, and
c) administering to the subject selected in step b) a drug treatment targeting liver fibrosis and/or its complications.

As used herein, a "treatment targeting liver fibrosis and/or its complications" may for instance be increased surveillance for liver cancer, increased surveillance for oesophageal varices, or drug treatment.
As used herein, "drug treatment" or "drug treatment targeting liver fibrosis and/or its complications" may for instance refer to treatment with a pancreatic lipase inhibitor, a PPARgamma agonist, a leptin analogue, a probiotic or a prebiotic.

The inventors demonstrated that subjects suffering from liver fibrosis displayed a significantly lower blood and feces bacterial diversity compared to healthy subjects, whatever the taxonomic level considered.

Accordingly, in particular embodiments, the methods disclosed herein involving a step of determination of bacterial diversity, further comprise a step pre-b) of comparing the bacterial diversity determined in step a) with a threshold value.

Said threshold value is preferably the value that gives a negative predictive value and a positive predictive value superior to 80%, preferably superior to 85%, more preferably superior to 90%, even more preferably superior to 95% in the targeted population. As used herein, the term "targeted population" refers to a population constituted of subjects who share certain biological parameters such as e.g. gender, age group, or certain environmental parameters such as e.g. geographical region.

Preferably, the comparison step involves determining whether the determined bacterial diversity is increased or decreased compared to the threshold value.

In preferred embodiments, a lower bacterial diversity determined in step a) compared to the threshold value indicates that the subject suffers from liver fibrosis.

Similarly, in particular embodiments, the methods disclosed herein involving a step of combination of proportions, further comprise a step pre-b) of comparing the combination value determined in step a2) with a threshold value. Said threshold value is preferably the value that gives a negative predictive value and a positive predictive value superior to 80%, preferably superior to 85%, more preferably superior to 90%, even more preferably superior to 95% in the targeted population. As used herein, the term "targeted population" refers to a population constituted of subjects who share certain biological parameters such as e.g. gender, age group, or certain environmental parameters such as e.g. geographical region.

Preferably, the comparison step involves determining whether the combination value is increased or decreased compared to the threshold value according to the invention.

In preferred embodiments, when the proportion of the *Variovorax* genera, and the proportions of bacteria belonging to the *Sphingomonas* genera, to the *Stenotrophomonas* genera, to the *Bosea* genera and to the *Micrococcus* genera are combined, an increased combination value determined in step a2) compared to the threshold value indicates that the subject suffers from liver fibrosis.

In preferred embodiments, when the proportion of the *Variovorax* genera, the proportions of bacteria belonging to the *Sphingomonas* genera, to the *Stenotrophomonas* genera, to the *Bosea* genera and to the *Micrococcus* genera and the total bacterial level are combined, an increased combination value determined in step a2) compared to the threshold value indicates that the subject suffers from liver fibrosis.

In preferred embodiments, when the proportion of the *Variovorax* genera, and the proportions of bacteria belonging to the *Proteobacteria* phylum and to the *Actinobacteria* phylum are combined, an increased combination value determined in step a2) compared to the threshold value indicates that the subject suffers from liver fibrosis.

In preferred embodiments, when the proportion of the *Variovorax* genera, the proportions of bacteria belonging to the *Proteobacteria* phylum and to the *Actinobacteria* phylum and the total bacterial level are combined, an increased combination value determined in step a2) compared to the threshold value indicates that the subject suffers from liver fibrosis.

Similarly, in particular embodiments, the methods involving a step of determination of the amount or relative proportion of bacterial gene functions, further comprise a step pre-b) of comparing the amount or relative proportion of bacterial gene functions from at least one metabolic pathway determined in step a) with a threshold value.

Said threshold value is preferably the value that gives a negative predictive value and a positive predictive value superior to 80%, preferably superior to 85%, more preferably superior to 90%, even more preferably superior to 95% in the targeted population. As used herein, the term "targeted population" refers to a population constituted of subjects who share certain biological parameters such as e.g. gender, age group, or certain environmental parameters such as e.g. geographical region.

Preferably, the comparison step involves determining whether the determined amount or relative proportion of bacterial genes functions from at least one metabolic pathway is increased or decreased compared to the threshold value.

In preferred embodiments, a lower amount or relative proportion of bacterial gene functions from at least one metabolic pathway, in particular one metabolic pathway selected from the group consisting of xenobiotics biodegradation and metabolism, metabolism of cofactors and vitamins and lipid metabolism, determined in step a) compared to the threshold value indicates that the subject suffers from liver fibrosis.

In other preferred embodiments, a higher amount or relative proportion of bacterial gene functions from the Nif family determined in step a) compared to the threshold value indicates that the subject suffers from liver fibrosis.

Preferably, when the determined bacterial diversity, combination value, amount of bacterial gene functions, or relative proportion of bacterial gene functions is increased compared to the threshold value, its value is significantly higher than the threshold value.

Also preferably, when the determined bacterial diversity, combination value, amount of bacterial gene functions or relative proportion of bacterial gene functions is decreased compared to the threshold value, its value is significantly lower than the threshold value.

*Methods of screening*

The disclosure also pertains to a method for screening a probiotic, a prebiotic, a chemical compound or a biological compound suitable for preventing and/or treating liver fibrosis, comprising the steps of:

A) determining bacterial diversity, as defined in the section *"Bacterial diversity"* above, in a biological sample, as defined in the section *"Biological sample"* above, from an obese subject, as defined in the section *"Subject"* above, suffering from liver fibrosis who has been treated with the candidate probiotic, prebiotic, chemical or biological compound, and
B) comparing said bacterial diversity with that of a control obese subject suffering from liver fibrosis who has not been treated with said candidate probiotic, prebiotic, chemical compound or biological compound, and
C) based on the result of the comparison at step B), determining if said candidate probiotic, prebiotic, chemical compound or biological compound is suitable for preventing and/or treating liver fibrosis.

The present disclosure also concerns a method for screening a probiotic, a prebiotic, a chemical compound or a biological compound suitable for preventing and/or treating liver fibrosis, said method comprising the steps of:

A1) determining the proportion of bacteria belonging to a particular taxon and the proportion of bacteria belonging to at least one other particular taxon, as defined in the section *"Bacterial diversity"* above, in a biological sample, as defined in the section *"Biological sample",* from an obese subject, as defined in the section *"Subject"* above, suffering from liver fibrosis who has been treated with the candidate probiotic, prebiotic, chemical or biological compound,
A2) combining the proportions determined in step A1), as defined in the section *"Combination"* above, to obtain a combination value,
B) comparing said combination value with that of a control obese subject suffering from liver fibrosis who has not been treated with said candidate probiotic, prebiotic, chemical compound or biological compound, and
C) based on the result of the comparison at step B), determining if said candidate probiotic, prebiotic, chemical compound or biological compound is suitable for preventing and/or treating liver fibrosis.

The disclosure also concerns a method for screening a probiotic, a prebiotic, a chemical compound or a biological compound suitable for preventing and/or treating liver fibrosis, comprising the steps of:

A) determining the amount or relative proportion of bacterial gene functions from at least one metabolic pathway, as defined in the section *"Bacterial gene functions"* above, in a biological sample, as defined in the section *"Biological sample"* above, from an obese subject, as defined in the section *"Subject"* above, suffering from liver fibrosis who has been treated with the candidate probiotic, prebiotic, chemical or biological compound, and
B) comparing said amount or relative proportion of bacterial gene functions with that of a control obese subject suffering from liver fibrosis who has not been treated with said candidate probiotic, prebiotic, chemical compound or biological compound, and
C) based on the result of the comparison at step B), determining if said candidate probiotic, prebiotic, chemical compound or biological compound is suitable for preventing and/or treating liver fibrosis.

As used herein, the term "probiotics" denotes dietary supplements and live microorganisms containing potentially beneficial bacteria or yeasts. According to the currently adopted definition by FAO/WHO, probiotics correspond to live microorganisms which when administered in adequate amounts confer a health benefit on the host. Examples of probiotics include bacterial strains of the genera *Bifidobacterium, Lactobacillus, Bacteroides* or of the class *Fusobacteria.*
As used herein, the term "prebiotics" denotes a non-digestible food ingredient that beneficially affects the host by selectively stimulating as a substrate the growth and/or activity of one or a limited number of bacteria in the intestine, in particular in the colon, and thus improves host health.
In the context of the disclosure, "prebiotics" encompass isolated or purified prebiotics as well as natural prebiotics present in dietary supplements.
In the context of the disclosure, "probiotics", encompass isolated or purified probiotics as well as natural probiotics present in dietary supplements.
As used herein, the term "chemical or biological compound" encompasses chemically synthetized compounds and compounds of biological origin which have an effect on the growth, metabolism, the survival of bacteria and/or their passage through the intestinal barrier. In particular, chemical or biological compounds according to the disclosure include molecules which modify the bacterial flora of the digestive tract and/or which modify the migration of bacteria through the digestive tract and/or which modify the permeability of the intestinal epithelial barrier. Examples of chemical or biological compounds include bactericides, antibiotics, as well as compounds acting on epithelial intercellular tight junctions, microvilli, cell coat, and/or intestinal epithelial cells.

The control subject which has not been treated may be a subject unrelated to the subject receiving the candidate prebiotic, probiotic or chemical or biological compound, or the same subject before treatment with the candidate prebiotic, probiotic or chemical or biological compound.

*Variovorax for treatment*

The present inventors particularly demonstrated that, among patients with high steatosis score, the presence of liver fibrosis correlates inversely with the blood level of *Variovorax* DNA. These results suggest that, among obese individuals, a high proportion of *Variovorax* bacteria in the blood protects against liver fibrosis.

Accordingly, another aspect of the disclosure pertains to bacteria belonging to the *Variovorax* genera for use as a probiotic, for preventing and/or treating liver fibrosis, as defined in the section *"Fibrosis"* above, in particular in obese subjects, as defined in the section *"Subject"* above.

In particular, the disclosure concerns bacterial belonging to the *Variovorax* genera for use for preventing and/or treating liver fibrosis, as defined in the section *"Fibrosis"* above, in particular in obese subjects, as defined in the section *"Subject"* above.

The present disclosure also concerns a method for preventing and/or treating liver fibrosis, as defined in the section *"Fibrosis"* above, in a subject, as defined in the section *"Subject"* above, in particular an obese subject, as defined in the section *"Subject"* above, comprising administering in a subject in need thereof a therapeutically effective amount of bacteria belonging to the *Variovorax* genera.

The present disclosure also concerns the use of bacteria belonging to the *Variovorax* genera in the manufacture of a medicament intended for the prevention and/or the treatment of liver fibrosis, as defined in the section *"Fibrosis"* above, in particular in an obese subject, as defined in the section *"Subject"* above.

Preferably, said bacteria belonging to the *Variovorax* genera are bacteria belonging to the *Variovorax paradoxus* species.

The present invention will be further illustrated by the above figures and example.

Brief description of the sequences

SEQ ID NO: 1 shows the sequence of the reference *Escherichia coli* 16S rDNA gene.
SEQ ID NO: 2 shows the sequence of the forward primer eubac-F.
SEQ ID NO: 3 shows the sequence of the reverse primer eubac-R.
SEQ ID NO: 4 shows the sequence of the primer noted Vaiomer 1F.
SEQ ID NO: 5 shows the sequence of the primer noted Vaiomer 1R.
SEQ ID NO: 6 shows the sequence of the primer noted Vaiomer 2F.
SEQ ID NO: 7 shows the sequence of the first part of the primer noted Vaiomer 2R before the index.
SEQ ID NO: 8 shows the sequence of the second part of the primer noted Vaiomer 2R after the index.
SEQ ID NO: 9 shows the sequence of the primer noted 28F.
SEQ ID NO: 10 shows the sequence of the primer noted 519R.

**Brief description of the figures**

**Figure 1:** Distribution of blood bacterial diversity (Shannon index) at different taxonomic levels in the study population of example 1.

**Figure 2:** Mean concentrations (bar plot) and individual values (dot plot) of bacterial diversity (Shannon index) in patients with or without liver fibrosis (* $p < 0.05$; ** $p < 0.01$) from example 1.

**Figure 3:** Mean relative proportions of bacterial phyla in blood of the overall study population of example 1.

**Figure 4:** Mean (bar plot) and individual values (dot plot) of relative proportions of relevant blood bacterial taxa in patients with or without liver fibrosis (* $p < 0.05$; ** $p < 0.01$) from example 1.

**Figure 5:** Repartition of patients with or without liver fibrosis depending on liver steatosis score and blood *Variovorax paradoxus* proportion assessed by 16S metagenomics sequencing.

**Figure 6:** Different multicomponent biomarkers were designed using either 16S qPCR values **(a),** 16S metagenomics data **(b, d)** or both **(c, e).** The performance of these biomarkers is presented on dot plots (left panels) showing the repartition in logit units for patients with or without liver fibrosis, and by ROC curve (left panels).

**Figure 7:** Mean relative proportions of bacterial phyla in feces of the overall study population.

**Figure 8:** Mean concentrations (bar plot) and individual values (dot plot) of relative proportions of relevant fecal bacterial taxa in patients with or without liver fibrosis (* $p < 0.05$; ** $p < 0.01$).

**Figure 9:** Linear regression of *Variovorax paradoxus* DNA blood level with several fecal taxa correlated or inversely correlated with liver fibrosis. Each dot represents a patient. All values are expressed in % of reads in 16S metagenomic sequencing. R is the Pearson correlation coefficient.

**Figures 10-12:** Functional impact of the microbiota modifications in patients of the discovery cohort with fibrosis of example 1.

**Figure 10:** Number of PICRUSt predicted bacterial gene functions with a relative abundance increased (light grey) or decreased (dark grey) at least 1.5 fold with fibrosis in three different metabolic pathways.

**Figure 11:** Same as in Figure 10 but taking into account only the significantly modified functions ($p < 0.05$ in Mann-Whitney test) with fibrosis.

**Figure 12:** PICRUSt predicted relative abundances of the gene functions of the Nif Family in control patients and patients with fibrosis.

## EXAMPLES

### Example 1

This example demonstrates that the level of bacterial diversity in blood and feces of obese subjects is useful marker of liver fibrosis. Furthermore, the inventors identified specific combinations of proportions of particular bacterial taxa and optionally total bacterial level enabling diagnosing liver fibrosis in obese subjects with high specificity and sensitivity.

Materials and methods

*Population*

The study was carried out in a subset (50 Spanish patients) of the Florinash cohort. Florinash is a cross sectional study on severely obese patients, well characterized with respect to the severity of the NAFLD. The primary aim of Florinash was to describe the relationship between intestinal microbiota and nonalcoholic steatohepatitis (NASH).

Inclusion criteria were age 40 to 60 years, body mass index (BMI) >40 kg/m$^2$, absence of any systemic disease, absence of clinical symptoms and signs of infection in the previous month by structured questionnaire to the patient. Exclusion criteria were serious chronic associated illness (heart failure, cirrhosis, panhypopituitarism or autoimmune diseases), consumption of alcohol (> 20 g ethanol intake per day) or use of medications able to interfere with insulin action. Among the 50 patients, 37 patients had buffy coat samples available and 44 had fecal samples available. All 50 patients underwent liver biopsies. The characteristics of the 37 patients with buffy coat and of the 50 patients of the overall population are summarized in **Table 1** and in **Table 2,** respectively.

**Table 1:** Characteristics of the study population with blood samples

| | ***Controls (No fibrosis)*** | | ***Cases (Fibrosis)*** | | **Cases vs controls** |
|---|---|---|---|---|---|
| **Total (n)** | **26** | | **11** | | |
| **Categorical variables** | **n** | **%** | **n** | **%** | **p (Fisher)** |
| **Men** | 5 | | 2 | | 1.00 |
| **Women Smoking status*** | 21 | | 9 | | 1.00 |
| **Never smoked** | 12 | 48.0 | 6 | 54.5 | 1.00 |
| **Former smoker** | 7 | 28.0 | 3 | 27.3 | |
| **Current smoker** | 6 | 24.0 | 2 | 18.2 | |
| **Treated hypertension** | 11 | 42.3 | 6 | 54.5 | 0.72 |
| **Treated diabetes** | 6 | 23.1 | 5 | 45.5 | 0.24 |

(continued)

| | Controls (No fibrosis) | | Cases (Fibrosis) | | Cases vs controls |
|---|---|---|---|---|---|
| **Total (n)** | **26** | | **11** | | |
| **Categorical variables** | **n** | **%** | **n** | **%** | **p (Fisher)** |
| **Treated dyslipidemia** | 6 | 23.1 | 2 | 18.2 | 1.00 |
| **Quantitative variables** | **Mean** | **SD** | **Mean** | **SD** | ***p* (Mann-Whitney)** |
| **Age (years)** | 46.2 | 8.9 | 48.1 | 9.3 | 0.56 |
| **Body mass index (kg/m$^2$)** | 44.7 | 6.7 | 41.9 | 6.5 | 0.17 |
| **Total cholesterol (mg/dl)** | 193.5 | 34.2 | 188.5 | 28.1 | 0.66 |
| **HDL cholesterol (mg/dl)** | 47.5 | 10.5 | 44.2 | 5.5 | 0.27 |
| **GPT (U/l)** | 25.2 | 19.3 | 24.4 | 6.5 | 0.17 |
| **GGT (U/l)** | 20.5 | 9.4 | 23.9 | 7.2 | 0.10 |
| **Glucose (mg/dl)** | 99.6 | 29.2 | 112.5 | 37.4 | 0.39 |
| **C reactive protein (mg/dl)*** | 0.73 | 0.44 | 0.78 | 0.75 | 0.57 |
| **Hematocrit (%)** | 40.5 | 4.6 | 38.6 | 4,0 | 0.33 |
| **Leukocyte (10$^3$/µl)** | 7.3 | 2,0 | 7.8 | 2,0 | 0.55 |
| **Neutrophil (10$^3$/µl)** | 4.6 | 1.9 | 4.9 | 1.9 | 0.64 |
| **Blood 16S DNA (copies/µl)** | 239.3 | 186.4 | 652.6 | 285.4 | 0.0002 |
| GPT: Glutamic-Pyruvic Transaminase, GGT: Gamma-glutamyl transpeptidase, HDL: High-density lipoprotein. *Data is missing for one patient. | | | | | |

**Table 2:** Characteristics of the total study population

| | Controls (No fibrosis) | | Cases (Fibrosis) | | Cases vs controls |
|---|---|---|---|---|---|
| **Total (n)** | **38** | | **12** | | |
| **Categorical variables** | **n** | **%** | **n** | **%** | ***p* (Fisher)** |
| **Men** | 6 | 15.8 | 3 | 25.0 | 0.67 |
| **Women Smoking status*** | 32 | 84.2 | 6 | 75.0 | 0.67 |
| Never smoked | 19 | 51.4 | 6 | 50.0 | 1.00 |
| Former smoker | 10 | 27.0 | 4 | 33.3 | |
| Current smoker | 8 | 21.6 | 2 | 16.7 | |
| **Treated hypertension** | 16 | 42.1 | 7 | 58.3 | 0.51 |
| **Treated diabetes** | 10 | 26.3 | 5 | 41.7 | 0.47 |
| **Treated dyslipidemia** | 8 | 21.1 | 2 | 16.7 | 1.00 |
| **Quantitative variables** | **Mean** | **SD** | **Mean** | **SD** | ***p* (Mann-Whitney)** |
| **Age (years)** | 47.16 | 8.6 | 47.75 | 9.0 | 0.81 |
| **Body mass index (kg/m$^2$)** | 45.2 | 6.7 | 42.4 | 6.5 | 0.17 |
| **Total cholesterol (mg/dl)** | 190.2 | 35.4 | 185.4 | 28.9 | 0.70 |
| **HDL cholesterol (mg/dl)** | 48.7 | 13.3 | 43.9 | 5.3 | 0.15 |
| **GPT (U/l)** | 26.4 | 18.5 | 30.1 | 20.8 | 0.17 |
| **GGT (U/l)** | 25.2 | 15.3 | 34.8 | 38.2 | 0.30 |
| **Glucose (mg/dl)** | 106.8 | 39.4 | 110.8 | 36.2 | 0.93 |
| **C reactive protein (mg/dl)*** | 0.83 | 0.64 | 0.82 | 0.73 | 0.59 |
| **Hematocrit (%)** | 40.4 | 4.2 | 38.9 | 4.0 | 0.43 |
| **Leukocyte (10$^3$/µl)** | 7.5 | 2.0 | 8.6 | 3.6 | 0.45 |
| **Neutrophil (10$^3$/µl)** | 4.6 | 1.7 | 6.0 | 4.0 | 0.37 |
| GPT: Glutamic-Pyruvic Transaminase, GGT: Gamma-glutamyl transpeptidase, HDL: High-density lipoprotein. *Data is missing for one patient. | | | | | |

*Liver biopsies and NAFLD diagnosis*

Patients provided written informed consent for the liver biopsies. The histological features of steatosis, inflammation (portal and lobular), hepatocyte ballooning and fibrosis were scored using the scoring system for NAFLD, as described in Angulo *et al.* (2007) *Hepatology* **45**:846-854. Features of steatosis, lobular inflammation and hepatocyte ballooning were combined in a score ranging from 0 to 8, named the NAFLD activity score (NAS). NAS 5 is diagnostic of non-alcoholic steatohepatitis, NAS 2 is diagnostic of simple steatosis and values in between are considered indeterminate.

*DNA extraction from buffy coat samples*

Total DNA was extracted from 100 μl of buffy coat samples using the NucleoSpin® Blood kit (MachereyNagel, Germany) after a mechanical lysis step of 2 times 30 sec at 20 Hz in a bead beater (TissueLyser, Qiagen, Netherlands) with 0.1 mm glass beads (MoBio, CA, USA). The quality and quantity of extracted nucleic acids were controlled by gel electrophoresis (1% w/w agarose in TBE 0.5x) and NanoDrop 2000 UV spectrophotometer (Thermo Scientific, MA, USA).

*16S rDNA quantitation by real-time qPCR*

The concentration of 16S rDNA copy number per 100 μl of buffy coat was evaluated by real-time qPCR using DNA-free Taq DNA Polymerase and primers EUBF 5'-TCCTACGGGAGGCAGCAGT-3' (SEQ ID NO: 2) and EUBR 5'-GGACTACCAGGGTATCTAATCCTGTT-3' (SEQ ID NO: 3) (HPLC grade, Eurogentec, Belgium). These primers target the V3-V4 hyper-variable regions of the 16S rDNA. The standard curve for 16S rDNA quantitation was performed by 10-fold dilution series of the complete 16S rDNA sequence of an *Escherichia coli* BL21 strain cloned in plasmids. Amplifications of samples and standard dilutions were performed in triplicates on a ViiATM 7 Real-Time PCR System (LifeTechnologies, CA, USA).

*16S rDNA sequence identification by MiSeq sequencing*

The V3-V4 hyper-variable regions of the 16S rDNA were amplified for MiSeq sequencing using a two-step PCR strategy. The first PCR was performed using DNA-free Taq DNA Polymerase, and primers Vaiomer 1F (CTTTCCCTACACGACG CTCTTCCGATCTTCCTACGGGAGGCAGCAGT, (SEQ ID NO: 4)) and Vaiomer 1R (GGAGTTCAGACGTGTGCTCTTCCGATCTGGACTACCAGGGTATCTAATCCTGTT (SEQ ID NO: 5)) which include the first part of the MiSeq P5/P7 adaptors. Sample multiplexing was performed using tailor-made 6 bases unique indices, which were added during the second PCR step at the same time as the second part of the P5/P7 adaptors with primers Vaiomer 2F (AATGATACGGCGACCACCGAGATCTACACTCTTTCCCTACACGAC (SEQ ID NO: 6)) and primer Vaiomer 2R (CAAGCAGAAGACGGCATACGAGAT(SEQ ID NO: 7)-index-GTGACTGGAGTTCAGACGTGT(SEQ ID NO: 8). Both PCR reactions were carried out on a Veriti Thermal Cycler (LifeTechnologies) followed by an amplicons purification using the magnetic beads Agencourt AMPure XP - PCR Purification (Beckman Coulter, CA, USA). The quality of a set of 12 amplicons was tested using Agilent DNA 7500 chips on a Bioanalyzer 2100 (Agilent Technologies, CA, USA). The region of the 16S rDNA to be sequenced has a length of 467 bp for a total amplicon length of 522 bp after PCR 1 and of 588 bp after PCR 2 (using the 16S rDNA of *Escherichia coli* as a reference). The pool diluted to 7 pM with 15% PhiX Control v3 mixture was sequenced with the Illumina MiSeq system using Illumina MiSeq Reagent Kit v3 (2x300 bp Paired-End Reads, 15 Gb output, Illumina, CA, USA). An average of 120,000 raw reads (60,000 reads pairs) was generated per sample.

*16S metagenomic bioinformatics pipeline*

The bioinformatics 16S metagenomics pipeline is based on the protocol published by Kozich et al. (2013) Appl. Environ. Microbiol. 79:5112-5120, with adjustments for specific difficulties presented by the analysis of blood. The raw MiSeq sequencing data were demultiplexed and FASTQ were generated using Illumina CASAVA v1.8.2. During the read pair joining step with FLASH v1.2.7, strict constraints were applied to join overlapping regions in order to reject low quality sequences. The analysis environment Mothur (v1.33.0) was used to align the sequences against the SILVA bacteria reference 16S alignment (v102). The PCR chimeras were screened as described in the MiSeq SOP using UCHIME v4.2 and removed. The OTU clustering was performed at 97% sequence identity and the taxonomic assignment is provided as calculated by the Naive Bayesian Classifier against the RDP rRNA training set (v9) with a minimum bootstrap confidence score of 80%. The sequences that were not assigned to the Bacteria domain were filtered out of the results. The output matrix containing the relative abundance of OTUs per sample were then processed with the LEfSe (linear discriminant analysis effect size) algorithm using an alpha cutoff of 0.05 and an effect size cutoff of 2.0.

*DNA extraction and 16S metagenomic sequencing from fecal samples*

Fecal total DNA was extracted as previously described in Serino et al. (2012) Gut 61:543-553. The whole 16S bacterial DNA V1-V3 region was targeted by the the primers 28F of sequence GAGTTTGATCNTGGCTCAG (SEQ ID NO: 9) and 519R of sequence GTNTTACNGCGGCKGCTG (SEQ ID NO: 10) and sequenced by pyrosequencing with the 454 FLX Roche technologies at Research and Testing Laboratory (Texas, USA). An average of 3,000 raw reads was generated per sample.

*Statistical analyses and predictive models construction*

Non parametric Mann - Whitney's tests and Fisher's exact tests were conducted on quantitative and categorical variables respectively using the software PRISM v6.05 (GraphPad software, CA, USA), with fibrosis status as independent variable - $p < 0.05$ was considered significant. Multicomponent statistical analyses were performed with the software environment R version 3.1.2. Briefly, the correlation of the 16S rDNA level (qPCR) and taxa proportions (16S metagenomic) on the fibrosis status was investigated using two types of logistic regression models: classical logistic regression and regularized logistic regression with lasso penalty. Indeed, to take into account the high dimensional data and multicollinearity, lasso logistic regression ("glmnet" R package) was used to construct sparse models containing the best predictors (variable selection). The penalty parameters were determined through leave-one-out cross validation. Subsets of predictors selected by the lasso penalty regression were then used in classical logistic regression to construct different models of multicomponent biomarkers. Finally, the performance of classifier systems obtained with the different models were evaluated with the receiver operating characteristic (ROC) curve, area under the curve (AUC) and 95% confidence intervals (CI) computed with 2000 stratified bootstrap replicates ("pROC" R package).

Results

*Patients with liver fibrosis have lower blood bacterial 16S rDNA diversity*

16S rDNA was analyzed by 16S targeted metagenomic sequencing and the Shannon index, which reflects the bacterial alpha diversity, was calculated at different taxonomic levels. The distribution of the patients according to Shannon index showed that two populations of patients with different taxa richness were observed **(Figure 1).** The mean Shannon index of patients with liver fibrosis was significantly lower ($0.003 \leq p \leq 0.049$ depending on the level) than control patients **(Figure 2)** and shows a tendency to decrease with the severity of the disease. The lower bacterial diversity observed in the blood of fibrosis patients could be linked to modified bacterial translocation from the gut; changes which could then go on to impact the liver.

*A specific taxonomic signature characterizes patients with liver fibrosis*

Next, a taxonomic assignment of the 16S rDNA bacterial sequences present in the blood of patients with or without fibrosis was performed. As shown in **Figure 3,** the sequences of the overall population of patients belonged mainly to Proteobacteria (87.9 %) and Actinobacteria (7.3%) phyla and to a lesser extent to Firmicutes (3.7 %) and Bacteroidetes (1.1 %) phyla.

Analysis of the 16S rDNA sequences using the LEfSe (linear discriminant analysis effect size) algorithm (disclosed for example in Segata et al. (2011) Genome Biology 12:R60) or the Mann-Whitney test **(Figure 4)** showed specific differences in the proportion of blood taxa depending on the presence or absence of liver fibrosis therefore defining a specific signature of liver fibrosis. It is noteworthy that the proportion of *Actinobacteria* was significantly decreased ($p = 0.006$) in patients with liver fibrosis whereas *Proteobacteria* increased in the same group ($p = 0.005$). Changes in several taxa belonging to these phyla also correlated significantly with the fibrosis status of the patient, including the genera *Sphingomonas* ($p = 0.034$), *Bosea* ($p = 0.041$) and *Variovorax* ($p= 0.023$). The variation of these different taxa also displayed a tendency to correlate with the severity of the disease. Furthermore, the biological characteristics of the *Variovorax* genus (being composed of only a few species with relatively high variability in their 16S rDNA sequences) allowed the assignment with high confidence of all corresponding reads to the species *Variovorax paradoxus.*

*The relationship between liver steatosis and Variovorax paradoxus blood level correlates with liver fibrosis*

Fibrosis status is significantly correlated with higher liver steatosis scores in these patients, however high steatosis scores occur in both patients with or without liver fibrosis. Interestingly, among patients with high steatosis scores (combined score value over 3), the presence of fibrosis correlates inversely with the blood level of *Variovorax paradoxus* DNA **(Figure 5).** The results suggest that, among obese individuals, a high proportion of *Variovorax paradoxus* in the blood protects against liver fibrosis and that the combination of both steatosis and a low level of *Variovorax paradoxus* triggers liver fibrosis.

*Multicomponent blood bacterial biomarkers are powerful predictors of liver fibrosis*

Multicomponent analyses were performed to identify combinations of blood bacterial biomarkers for the presence of liver fibrosis **(Figure 6).** The "logit" values mentioned on **Figure 6,** which correspond to the following formula:

$$\text{logit} = \log\left(\frac{p}{1-p}\right)$$

with $p$ = probability to have fibrosis,
were maximized for each combination by the respective formulae cited below.

The inventors first evaluated the predictive value of blood 16S rDNA qPCR measurements alone **(Figure 6a)** by constructing a ROC curve with bootstrap validation to calculate the variance, CI and AUC. The 16S rDNA qPCR assay gave an AUC value of 0.87 (95% CI 0.72-0.88), using the formula : logit = $6.5 \times 10^{-5} \times$ [qPCR]- 3.6 , to maximize the "logit" value.

Methods of logistic regression and lasso penalized logistic regression were then used to construct multicomponent biomarkers with several sequenced taxa either at the genus level alone **(Figure 6b-c)** or using several taxonomic levels **(Figure 6d-e).** The ROC curve AUC (with bootstrap validation), positive predictive value (PPV) and negative predictive value (NPV) were calculated using the blood 16S metagenomics data alone **(Figure 6b,d)** or by combining metagenomic and qPCR data **(Figure 6c,e).**

The best combination of bacterial genera shouwed an AUC of 0.93 (95% CI0.83-0.93), using five different genera including *Variovorax, Sphingomonas, Bosea, Stenotophomonas* and *Micrococcus,* and using the formula :

$$\text{logit} = 11.1 \times \%\text{Sphingomonas} - 71.4 \times \%\text{Variovorax} - 29.7 \times \%\text{Stenotrophomonas} + 37.3 \times \%\text{Bosea} - 214.7 \times \%\text{Micrococcus} - 1.5$$

to maximize the "logit" value. Adding the qPCR data to these five metagenomics taxa increase the AUC of the multicomponent biomarker to 0.99 (95% CI0.97-1), using the formula :

$$\text{logit} = 16.2 \times \%\text{Sphingomonas} - 134.6 \times \%\text{Variovorax} - 163.4 \times \%\text{Stenotrophomonas} + 77.3 \times \%\text{Bosea} - 482.6 \times \%\text{Micrococcus} - 1.9\times10^{-4} \times [\text{qPCR}] - 5.3$$

to maximize the "logit" value.

If several taxonomic levels are used to design the multicomponent biomarker based on the metagenomics data, only three different taxa (the phyla *Actionobacteria* and *Proteobacteria* and the genus *Variovorax)* are sufficient to obtain an AUC of 0.90 (95% CI 0.78-0.91) using the formula :

$$\text{logit} = 14.4 \times \%\text{Proteobacteria} - 37.1 \times \%\text{Actinobacteria} - 76.3 \times \%\text{Variovorax} - 8.9$$

to maximize the "logit" value. The combination of the qPCR data with these taxa dramatically improved the sensitivity and specificity of the biomarkers to diagnose liver fibrosis achieving an AUC value of 1 (95% CI 1-1) (Figure 6e), using the formula :

$$\text{logit} = 29.5 \times \%\text{Proteobacteria} - 186.1 \times \%\text{Actinobacteria} - 411.4 \times \%\text{Variovorax} - 3.0\times10^{-4} \times [\text{qPCR}] - 13.7$$

to maximize the "logit" value.

*The compositions of fecal and blood microbiota are different*

To assess whether fecal microbiota was also modified in liver fibrosis, and how it differed from blood microbiota, 16S metagenomic sequencing of the 44 patients with available fecal samples were performed. The results showed that the composition of fecal microbiota and blood microbiota are totally different. Indeed, whereas as shown in **Figure 3,** blood microbiota is mainly composed of *Proteobacteria* and *Actinobacteria* phyla, fecal samples are composed of bacteria belonging mostly to the *Firmicutes* and *Bacteroidetes* phyla **(Figure 7).**

*The variations of specific taxa of gut microbiota correlate with liver fibrosis*

It was then aimed to identify differences in the composition of fecal microbiota taxa in patients with or without liver fibrosis. Correlations between the fibrosis status and changes in the proportion of taxa were characterized using the LEfSe algorithm or the Mann-Whitney test **(Figure 8).** Both analyses showed that the mean proportion of several fecal bacterial taxa varied between groups of patients. *Firmicutes* ($p = 0.002$) and *Actinobacteria* ($p = 0.006$) were significantly decreased in fibrosis, whereas *Bacteroidetes* ($p = 0.090$) and *Fusobacteria* ($p = 0.003$) were increased. Within these phyla, several taxa were also significantly modified in patients with liver fibrosis **(Figure 8),** in particular the families of *Ruminococcaceae* ($p = 0.019$) and *Lachnospiraceae* ($p = 0.004$), *Coriobacteriaceae* ($p = 0.017$) and *Fusobacteriaceae* ($p = 0.011$). The *Actinobacteria* phyla was decreased in both feces and blood, but the orders of bacteria within this phylum was different between the blood (*Actinomycetales*) and feces (*Coriobacteriales*) as shown in **Figure 4** and **Figure 8.** Altogether these data demonstrate that the gut microbiota was deeply impacted in patients with liver fibrosis.

*The variation of blood Variovorax paradoxus correlates with specific fecal taxa*

Since correlations involving different taxa between liver fibrosis and both fecal and blood microbiota were observed, it was then investigated whether there were correlations between fecal and blood taxa. Correlation plots and linear regression between taxa in blood and feces **(Figure 9)** showed that this type of correlation existed and that blood *Variovorax paradoxus* was the blood taxa which correlated the most with several fecal taxa (Pearson correlation coefficient, $|R|$, between 0.417 and 0.509). Interestingly these fecal taxa are themselves correlated with fibrosis **(Figure 8).** These results show the existence of an inter-correlation between elements of the blood and fecal microbiota with liver fibrosis, despite the observation that the modification of the blood microbiota is not the mirror of the modification of the fecal microbiota.

These results thus show that the level of bacterial diversity in blood and feces of obese subjects is useful marker of liver fibrosis. Furthermore, the inventors identified specific combinations of proportions of particular bacterial taxa and optionally total bacterial level enabling diagnosing liver fibrosis in obese subjects with high specificity and sensitivity.

## Example 2

This example demonstrates that determining the amount of bacterial gene functions from several metabolic pathways is useful for diagnosing liver fibrosis in obese patients.

Materials and methods

The PICRUSt software, disclosed in Langille et al. (2013) Nat. Biotechnol. 31:814-821, was applied on the sequencing data generated in Example 1.

Briefly, the PICRUSt software is a computational approach enabling predicting the functional composition of a metagenome using marker gene data and a database of reference genomes. PICRUSt uses an extended ancestral-state reconstruction algorithm to predict which gene families are present and then combines gene families to estimate the composite metagenome.

Results

*Prediction of metagenome functional content modifications in patients with liver fibrosis*

The inventors used the software PICRUSt (Phylogenetic Investigation of Communities by Reconstruction of Unobserved States) (Langille et al. (2013) Nat. Biotechnol. 31:814-82) to predict from the 16S metagenomic data the metagenome functional content (bacterial genes) present in the blood of the patients. A total of 5,817 different bacterial gene functions were predicted by PICRUSt, and among them 526 were significantly modified ($p < 0.05$) between groups of patients with or without liver fibrosis.

**Figures 10 and 11** illustrate some of the metabolic pathways containing numerous gene functions that are mostly decreased in the fibrosis group. **Figure 12** shows the detail of predicted relative abundance of functions of the Nif family, which are increased with liver fibrosis.

Accordingly, PICRUSt analysis revealed that, with the decrease of bacterial diversity, many blood microbiota functions, including xenobiotics biodegradation, are proportionally decreased in patients with fibrosis. On the other hand, the proportion of genes from other pathways such as nitrogenase of the Nif family is significantly increased in fibrosis.

SEQUENCE LISTING

<110> VAIOMER

<120> Method for diagnosing hepatic fibrosis based on bacterial profile and diversity

<130> BET16P2266

<150> EP15306606.3
<151> 2015-10-09

<160> 10

<170> PatentIn version 3.5

<210> 1
<211> 1542
<212> DNA
<213> Escherichia coli

<400> 1

```
aaattgaaga gtttgatcat ggctcagatt gaacgctggc ggcaggccta acacatgcaa      60
gtcgaacggt aacaggaagc agcttgctgc tttgctgacg agtggcggac gggtgagtaa     120
tgtctgggaa actgcccgat ggagggggat aactactgga aacggtagct aataccgcat     180
aacgtcgcaa gaccaaagag ggggaccttc gggcctcttg ccatcggatg tgcccagatg     240
ggattagcta gtaggtgggg taacggctca cctaggcgac gatccctagc tggtctgaga     300
ggatgaccag ccacactgga actgagacac ggtccagact cctacgggag gcagcagtgg     360
ggaatattgc acaatgggcg caagcctgat gcagccatgc cgcgtgtatg aagaaggcct     420
tcgggttgta aagtactttc agcggggagg aagggagtaa agttaatacc tttgctcatt     480
gacgttaccc gcagaagaag caccggctaa ctccgtgcca gcagccgcgg taatacggag     540
ggtgcaagcg ttaatcggaa ttactgggcg taaagcgcac gcaggcggtt tgttaagtca     600
gatgtgaaat ccccgggctc aacctgggaa ctgcatctga tactggcaag cttgagtctc     660
gtagaggggg gtagaattcc aggtgtagcg gtgaaatgcg tagagatctg gaggaatacc     720
ggtggcgaag gcggccccct ggacgaagac tgacgctcag gtgcgaaagc gtggggagca     780
aacaggatta gataccctgg tagtccacgc cgtaaacgat gtcgacttgg aggttgtgcc     840
cttgaggcgt ggcttccgga gctaacgcgt taagtcgacc gcctggggag tacggccgca     900
aggttaaaac tcaaatgaat tgacgggggc ccgcacaagc ggtggagcat gtggtttaat     960
tcgatgcaac gcgaagaacc ttacctggtc ttgacatcca cggaagtttt cagagatgag    1020
aatgtgcctt cgggagccgt gagacaggtg ctgcatggct gtcgtcagct cgtgttgtga    1080
aatgttgggt taagtcccgc aacgagcgca acccttatcc tttgttgcca gcggtccggc    1140
cgggaactca aaggagactg ccagtgataa actggaggaa ggtggggatg acgtcaagtc    1200
atcatggccc ttacgaccag ggctacacac gtgctacaat ggcgcataca aagagaagcg    1260

acctcgcgag agcaagcgga cctcataaag tgcgtcgtag tccggattgg agtctgcaac    1320
tcgactccat gaagtcggaa tcgctagtaa tcgtggatca gaatgccacg gtgaatacgt    1380
tcccgggcct tgtacacacc gcccgtcaca ccatgggagt gggttgcaaa agaagtaggt    1440
agcttaacct tcgggagggc gcttaccact ttgtgattca tgactggggt gaagtcgtaa    1500
caaggtaacc gtaggggaac ctgcggttgg atcacctcct ta                      1542
```

<210> 2
<211> 19
<212> DNA
<213> Artificial Sequence

<220>
<223> primer eubac-F

<400> 2
tcctacggga ggcagcagt 19

<210> 3
<211> 26
<212> DNA
<213> Artificial Sequence

<220>
<223> Primer eubac-R

<400> 3
ggactaccag ggtatctaat cctgtt 26

<210> 4
<211> 47
<212> DNA
<213> Artificial Sequence

<220>
<223> primer Vaiomer 1F

<400> 4
ctttccctac acgacgctct tccgatcttc ctacgggagg cagcagt 47

<210> 5
<211> 54
<212> DNA
<213> Artificial Sequence

<220>
<223> primer Vaiomer 1R

<400> 5
ggagttcaga cgtgtgctct tccgatctgg actaccaggg tatctaatcc tgtt 54

<210> 6
<211> 45
<212> DNA
<213> Artificial Sequence

<220>
<223> primer Vaiomer 2F

<400> 6
aatgatacgg cgaccaccga gatctacact ctttccctac acgac 45

<210> 7
<211> 24
<212> DNA
<213> Artificial Sequence

<220>
<223> first part of the primer Vaiomer 2R before the index.

<400> 7
caagcagaag acggcatacg agat 24

<210> 8
<211> 21
<212> DNA
<213> Artificial Sequence

<220>
<223> second part of the primer Vaiomer 2R after the index.

<400> 8
gtgactggag ttcagacgtg t 21

<210> 9
<211> 19
<212> DNA
<213> Artificial Sequence

<220>
<223> primer 28F

<220>
<221> misc_feature
<222> (11)..(11)
<223> n is a, c, g, or t

<400> 9
gagtttgatc ntggctcag 19

<210> 10
<211> 18
<212> DNA
<213> Artificial Sequence

<220>
<223> Primer 519R

<220>
<221> misc_feature
<222> (3)..(3)
<223> n is a, c, g, or t

<220>
<221> misc_feature
<222> (8)..(8)
<223> n is a, c, g, or t

<400> 10
gtnttacngc ggckgctg 18

## Claims

**1.** An *in vitro* method for diagnosing liver fibrosis in an obese subject, selecting an obese subject for liver biopsy, or for selecting an obese subject for treatment regimen targeting liver fibrosis and/or its complications, said method comprising the steps of:

a) determining bacterial diversity in a blood or feces sample from the subject by

(i) determining the Shannon index, or

(ii)

a1) determining the proportion of *Variovorax* genera, the proportion of bacteria belonging to the *Sphingomonas* genera, the proportion of bacteria belonging to the *Stenotrophomonas* genera, the proportion of bacteria belonging to the *Bosea* genera, and the proportion of bacteria belonging to the *Micrococcus* genera, with relation to the total bacterial level, and optionally the total bacterial level, in a blood or feces sample of said subject, and
a2) combining the proportions, and optionally the total bacterial level, determined in step a1) to obtain a combination value,
or

(iii)

a1) determining the proportion of *Variovorax* genera, the proportion of bacteria belonging to the *Proteobacteria* phylum and the proportion of bacteria belonging to the *Actineobacteria* phylum, with relation to the total bacterial level, and optionally the total bacterial level, in a blood or feces sample of said subject, and
a2) combining the proportions, and optionally the total bacterial level, determined in step a1) to obtain a combination value,

pre-b) comparing the bacterial diversity determined in step a)(i) or the combination value determined in step (ii) a2) or in step (iii) a2) with a threshold value, said threshold value being the value that gives a negative predictive value and a positive predictive value superior to 80% in the targeted population, and
b) based on the result of the bacterial diversity determination in step a)(i) or on the combination value obtained in step (ii) a2) or in step (iii) a2), diagnosing liver fibrosis in the subject, selecting the subject to undergo liver biopsy or selecting the subject to undergo treatment regimen targeting liver fibrosis and/or its complications,

wherein a lower bacterial diversity determined in step a) (i) or a higher combination value determined in step (ii) a2) or in step (iii) a2), compared to the threshold value indicates that the subject suffers from liver fibrosis, the subject can be selected to undergo liver biopsy or the subject can be selected to undergo treatment regimen targeting liver fibrosis and/or its complications.

**2.** An *in vitro* method for determining if an obese patient suffering from liver fibrosis and/or its complications responds to a drug treatment targeting liver fibrosis and/or its complications, said method comprising the steps of:

a) determining bacterial diversity in a blood or feces sample from the subject before drug treatment by

(i) determining the Shannon index, or
(ii)

a1) determining the proportion of *Variovorax* genera, the proportion of bacteria belonging to the *Sphingomonas* genera, the proportion of bacteria belonging to the *Stenotrophomonas* genera, the proportion of bacteria belonging to the *Bosea* genera, and the proportion of bacteria belonging to the *Micrococcus* genera, with relation to the total bacterial level, and optionally the total bacterial level, in a blood or feces sample from the subject before drug treatment, and
a2) combining the proportions, and optionally the total bacterial level, determined in step a1) to obtain a combination value before treatment;
or

(iii)

a1) determining the proportion of *Variovorax* genera, the proportion of bacteria belonging to the *Proteobacteria* phylum and the proportion of bacteria belonging to the *Actineobacteria* phylum, with relation to the total bacterial level, and optionally the total bacterial level, in a blood or feces sample of said subject before drug treatment, and
a2) combining the proportions, and optionally the total bacterial level, determined in step a1) to obtain a combination value before treatment,

a') determining bacterial diversity in a blood or feces sample from the subject after drug treatment by

(i) determining the Shannon index,or

(ii)

a'1) determining the proportion of *Variovorax* genera, the proportion of bacteria belonging to the *Sphingomonas* genera, the proportion of bacteria belonging to the *Stenotrophomonas* genera, the proportion of bacteria belonging to the *Bosea* genera, and the proportion of bacteria belonging to the *Micrococcus* genera, with relation to the total bacterial level, and optionally the total bacterial level, in a blood or feces sample from the subject after drug treatment, and

a'2) combining the proportions, and optionally the total bacterial level, determined in step a1) to obtain a combination value after treatment;

or

(iii)

a'1) determining the proportion of *Variovorax* genera, the proportion of bacteria belonging to the *Proteobacteria* phylum and the proportion of bacteria belonging to the *Actineobacteria* phylum, with relation to the total bacterial level, and optionally the total bacterial level, in a blood or feces sample of said subject after drug treatment, and

a'2) combining the proportions, and optionally the total bacterial level, determined in step a1) to obtain a combination value after treatment,

and

b) based on the result of the bacterial diversity determinations in steps a)(i) and a')(i) or on the combination values determined in steps (ii) a2) or (iii) a2) and (ii) a'2) or (iii) a'2), determining if the patient responds to said drug treatment.

**3.** A method for screening a probiotic, a prebiotic, a chemical compound or a biological compound suitable for preventing and/or treating liver fibrosis, comprising the steps of:

A) determining bacterial diversity in a blood or feces sample from an obese subject suffering from liver fibrosis who has been treated with the candidate probiotic, prebiotic, chemical or biological compound, by

(i) determining the Shannon index, or

(ii)

A1) determining the proportion of *Variovorax* genera, the proportion of bacteria belonging to the *Sphingomonas* genera, the proportion of bacteria belonging to the *Stenotrophomonas* genera, the proportion of bacteria belonging to the *Bosea* genera, and the proportion of bacteria belonging to the *Micrococcus* genera, with relation to the total bacterial level, and optionally the total bacterial level, in a blood or feces sample from an obese subject suffering from liver fibrosis who has been treated with the candidate probiotic, prebiotic, chemical or biological compound, and

A2) combining the proportions, and optionally the total bacterial level, determined in step A1) to obtain a combination value,

or

(iii)

A1) determining the proportion of *Variovorax* genera, the proportion of bacteria belonging to the *Proteobacteria* phylum and the proportion of bacteria belonging to the *Actineobacteria* phylum, with relation to the total bacterial level, and optionally the total bacterial level, in a blood or feces sample from an obese subject suffering from liver fibrosis who has been treated with the candidate probiotic, prebiotic, chemical or biological compound, and

A2) combining the proportions, and optionally the total bacterial level, determined in step A1) to obtain a combination value

and

B) comparing said bacterial diversity or said combination value with that of a control obese subject suffering from liver fibrosis who has not been treated with said candidate probiotic, prebiotic, chemical compound or biological compound, and
C) based on the result of the comparison at step B), determining if said candidate probiotic, prebiotic, chemical compound or biological compound is suitable for preventing and/or treating liver fibrosis.

**Patentansprüche**

1. *In-vitro*-Verfahren zur Diagnose von Leberfibrose bei einem adipösen Patienten, Auswahl eines adipösen Patienten für eine Leberbiopsie oder zur Auswahl eines adipösen Patienten für ein Behandlungsschema, das auf Leberfibrose und/oder ihre Komplikationen abzielt, wobei das Verfahren die folgenden Schritte umfasst:

    a) Bestimmen der bakteriellen Vielfalt in einer Blut- oder Stuhlprobe des Probanden durch

        (i) Bestimmen des Shannon-Index, oder
        (ii)

            a1) Bestimmen des Anteils der Gattung *Variovorax,* des Anteils der Bakterien der Gattung *Sphingomonas,* des Anteils der Bakterien der Gattung *Stenotrophomonas,* des Anteils der Bakterien der Gattung *Bosea,* und des Anteils der Bakterien der Gattung *Micrococcus,* in Bezug auf den Gesamtbakteriengehalt, und gegebenenfalls des Gesamtbakteriengehalts, in einer Blut- oder Stuhlprobe des Patienten, und
            a2) Kombinieren der in Schritt a1) bestimmten Anteile, und gegebenenfalls des Gesamtbakteriengehalts, um einen Kombinationswert zu erhalten,
            oder

        (iii)

            a1) Bestimmen des Anteils der Gattung *Variovorax,* des Anteils der Bakterien des Phylums *Proteobacteria,* des Anteils der Bakterien des Phylums *Actineobacteria,* in Bezug auf den Gesamtbakteriengehalt, und gegebenenfalls des Gesamtbakteriengehalts, in einer Blut- oder Stuhlprobe des Patienten, und
            a2) Kombinieren der in Schritt a1) bestimmten Anteile, und gegebenenfalls des Gesamtbakteriengehalts, um einen Kombinationswert zu erhalten,

    vor b) Vergleichen der in Schritt a)(i) bestimmten bakteriellen Vielfalt oder des in Schritt (ii) a2) oder in Schritt (iii) a2) bestimmten Kombinationswertes mit einem Schwellenwert, wobei der Schwellenwert der Wert ist, der in der Zielpopulation einen negativen Vorhersagewert und einen positiven Vorhersagewert von mehr als 80 % ergibt, und
    b) basierend auf dem Ergebnis der Bestimmung der bakteriellen Vielfalt in Schritt a)(i) oder auf dem in Schritt (ii) a2) oder in Schritt (iii) a2) erhaltenen Kombinationswert, die Diagnose der Leberfibrose bei dem Patienten, die Auswahl des Patienten, der einer Leberbiopsie unterzogen werden soll, oder die Auswahl des Patienten, der einem Behandlungsschema, das auf die Leberfibrose und/oder ihre Komplikationen abzielt, unterzogen werden soll,

    wobei eine in Schritt a) (i) bestimmte geringere bakterielle Vielfalt oder ein in Schritt (ii) a2) oder in Schritt (iii) a2) bestimmter höherer Kombinationswert, verglichen mit dem Schwellenwert, anzeigt, dass der Patient an Leberfibrose leidet, der Patient zur Durchführung einer Leberbiopsie ausgewählt werden kann oder der Patient zur Durchführung eines Behandlungsregimes ausgewählt werden kann, das auf Leberfibrose und/oder deren Komplikationen abzielt.

2. *In-vitro*-Verfahren zum Bestimmen, ob ein adipöser Patient, der an Leberfibrose und/oder deren Komplikationen leidet, auf eine Arzneimittelbehandlung anspricht, die auf Leberfibrose und/oder deren Komplikationen abzielt, wobei das Verfahren die folgenden Schritte umfasst:

    a) Bestimmen der Bakterienvielfalt in einer Blut- oder Stuhlprobe des Patienten vor der Arzneimittelbehandlung durch

(i) Bestimmen des Shannon-Index, oder

(ii)

a1) Bestimmen des Anteils der Gattung *Variovorax,* des Anteils der Bakterien der Gattung *Sphingomonas,* des Anteils der Bakterien der Gattung *Stenotrophomonas,* des Anteils der Bakterien der Gattung *Bosea,* und des Anteils der Bakterien der Gattung *Micrococcus,* in Bezug auf den Gesamtbakteriengehalt, und gegebenenfalls des Gesamtbakteriengehalts, in einer Blut- oder Stuhlprobe des Patienten vor der Arzneimittelbehandlung, und

a2) Kombinieren der in Schritt a1) bestimmten Anteile, und gegebenenfalls des Gesamtbakteriengehalts, um einen Kombinationswert vor der Behandlung zu erhalten; oder

(iii)

a1) Bestimmen des Anteils der Gattung *Variovorax,* des Anteils der Bakterien des Phylums *Proteobacteria,* des Anteils der Bakterien des Phylums *Actineobacteria,* in Bezug auf den Gesamtbakteriengehalt, und gegebenenfalls des Gesamtbakteriengehalts, in einer Blut- oder Stuhlprobe des Patienten vor der Arzneimittelbehandlung, und

a2) Kombinieren der in Schritt a1) bestimmten Anteile, und gegebenenfalls des Gesamtbakteriengehalts, um einen Kombinationswert vor der Behandlung zu erhalten,

a') Bestimmen der Bakterienvielfalt in einer Blut- oder Stuhlprobe des Patienten nach der Arzneimittelbehandlung durch

(i) Bestimmen des Shannon-Index, oder

(ii)

a'1) Bestimmen des Anteils der Gattung *Variovorax,* des Anteils der Bakterien der Gattung *Sphingomonas,* des Anteils der Bakterien der Gattung *Stenotrophomonas,* des Anteils der Bakterien der Gattung *Bosea,* und des Anteils der Bakterien der Gattung *Micrococcus,* in Bezug auf den Gesamtbakteriengehalt, und gegebenenfalls des Gesamtbakteriengehalts, in einer Blut- oder Stuhlprobe des Patienten nach der Arzneimittelbehandlung, und

a'2) Kombinieren der in Schritt a1) bestimmten Anteile, und gegebenenfalls des Gesamtbakteriengehalts, um einen Kombinationswert nach der Behandlung zu erhalten; oder

(iii)

a'1) Bestimmen des Anteils der Gattung *Variovorax,* des Anteils der Bakterien des Phylums *Proteobacteria,* des Anteils der Bakterien des Phylums *Actineobacteria,* in Bezug auf den Gesamtbakteriengehalt, und gegebenenfalls des Gesamtbakteriengehalts, in einer Blut- oder Stuhlprobe des Patienten nach der Arzneimittelbehandlung, und

a'2) Kombinieren der in Schritt a1) bestimmten Anteile, und gegebenenfalls des Gesamtbakteriengehalts, um einen Kombinationswert nach der Behandlung zu erhalten,
und

b) basierend auf dem Ergebnis der Bestimmungen der bakteriellen Vielfalt in den Schritten a)(i) und a')(i) oder auf den Kombinationswerten, die in den Schritten (ii) a2) oder (iii) a2) und (ii) a'2) oder (iii) a'2) bestimmt wurden, zu bestimmen, ob der Patient auf die genannte Arzneimittelbehandlung anspricht.

**3.** Verfahren zum Screenen eines Probiotikums, eines Präbiotikums, einer chemischen Verbindung oder einer biologischen Verbindung, die zur Prävention und/oder Behandlung von Leberfibrose geeignet sind, folgende Schritte umfassend:

A) Bestimmung der bakteriellen Vielfalt in einer Blut- oder Stuhlprobe eines an Leberfibrose erkrankten adipösen Patienten, der mit der in Frage kommenden probiotischen, präbiotischen, chemischen oder biologischen Verbindung behandelt wurde, durch

(i) Bestimmen des Shannon-Index, oder

(ii)

A1) Bestimmen des Anteils der Gattung *Variovorax,* des Anteils der Bakterien der Gattung *Sphingomonas,* des Anteils der Bakterien der Gattung *Stenotrophomonas,* des Anteils der Bakterien der Gattung *Bosea,* und des Anteils der Bakterien der Gattung *Micrococcus,* in Bezug auf den Gesamtbakteriengehalt, und gegebenenfalls des Gesamtbakteriengehalts, in einer Blut- oder Stuhlprobe eines an Leberfibrose leidenden adipösen Patienten, der mit der in Frage kommenden probiotischen, präbiotischen, chemischen oder biologischen Verbindung behandelt wurde, und
A2) Kombinieren der in Schritt A1) bestimmten Anteile, und gegebenenfalls des Gesamtbakteriengehalts, um einen Kombinationswert zu erhalten,
oder

(iii)

A1) Bestimmen des Anteils der Gattung *Variovorax,* des Anteils der Bakterien des Phylums *Proteobacteria,* des Anteils der Bakterien des Phylums *Actineobacteria,* in Bezug auf den Gesamtbakteriengehalt, und gegebenenfalls des Gesamtbakteriengehalts, in einer Blut- oder Stuhlprobe eines an Leberfibrose leidenden adipösen Patienten, der mit der in Frage kommenden probiotischen, präbiotischen, chemischen oder biologischen Verbindung behandelt wurde, und
A2) Kombinieren der in Schritt A1) bestimmten Anteile, und gegebenenfalls des Gesamtbakteriengehalts, um einen Kombinationswert zu erhalten
und

B) Vergleichen der bakteriellen Vielfalt oder des Kombinationswertes mit demjenigen eines an Leberfibrose leidenden adipösen Kontroll-Patienten, der nicht mit der in Frage kommenden probiotischen, präbiotischen, chemischen oder biologischen Verbindung behandelt wurde, und
C) Bestimmen, basierend auf dem Ergebnis des Vergleichs in Schritt B), ob die in Frage kommende probiotische, präbiotische, chemische oder biologische Verbindung zur Prävention und/oder Behandlung von Leberfibrose geeignet ist.

## Revendications

**1.** Procédé *in vitro* de diagnostic la fibrose hépatique chez un sujet obèse, de sélection d'un sujet obèse pour une biopsie hépatique, ou de sélection d'un sujet obèse pour un régime thérapeutique ciblant la fibrose hépatique et/ou ses complications, ledit procédé comprenant les étapes consistant à :

a) déterminer la diversité bactérienne dans un échantillon de sang ou de fèces du sujet en

(i) déterminant l'indice de Shannon, ou
(ii)

a1) déterminant la proportion du genre *Variovorax,* la proportion de bactéries appartenant au genre *Sphingomonas,* la proportion de bactéries appartenant au genre *Stenotrophomonas,* la proportion de bactéries appartenant au genre *Bosea,* et la proportion de bactéries appartenant au genre *Micrococcus,* par rapport au niveau bactérien total, et éventuellement le niveau bactérien total, dans un échantillon de sang ou de fèces dudit sujet, et
a2) combinant les proportions, et éventuellement le niveau bactérien total, déterminés à l'étape a1) afin d'obtenir une valeur de combinaison, ou

(iii)

a1) déterminant la proportion du genre *Variovorax,* la proportion de bactéries appartenant au phylum des *Proteobacteria* et la proportion de bactéries appartenant au phylum des *Actineobacteria,* par rapport au niveau bactérien total, et éventuellement le niveau bactérien total, dans un échantillon de sang ou de fèces dudit sujet, et
a2) combinant les proportions, et éventuellement le niveau bactérien total, déterminés à l'étape a1) afin d'obtenir une valeur de combinaison,

pré-b) comparer la diversité bactérienne déterminée à l'étape a)(i) ou la valeur de combinaison déterminée à l'étape (ii) a2) ou à l'étape (iii) a2) avec une valeur seuil, ladite valeur seuil étant la valeur qui permet d'obtenir une valeur prédictive négative et une valeur prédictive positive supérieures à 80 % dans la population ciblée, et b) en se basant sur le résultat de la détermination de la diversité bactérienne à l'étape a)(i) ou sur la valeur de combinaison obtenue à l'étape (ii) a2) ou à l'étape (iii) a2), diagnostiquer la fibrose hépatique chez le sujet, sélectionner le sujet pour qu'il soit soumis à une biopsie hépatique ou sélectionner le sujet pour qu'il reçoive un régime thérapeutique ciblant la fibrose hépatique et/ou ses complications,

dans lequel une diversité bactérienne plus faible déterminée à l'étape a) (i) ou une valeur de combinaison plus élevée déterminée à l'étape (ii) a2) ou à l'étape (iii) a2), par comparaison à la valeur seuil, indique que le sujet souffre de fibrose hépatique, que le sujet peut être sélectionné pour être soumis à une biopsie hépatique ou que le sujet peut être sélectionné pour recevoir un régime thérapeutique ciblant la fibrose hépatique et/ou ses complications.

**2.** Procédé *in vitro* pour déterminer si un patient obèse souffrant de fibrose hépatique et/ou ses complications répond à un traitement médicamenteux ciblant la fibrose hépatique et/ou ses complications, ledit procédé comprenant les étapes consistant à :

a) déterminer la diversité bactérienne dans un échantillon de sang ou de fèces du sujet avant le traitement médicamenteux en

(i) déterminant l'indice de Shannon, ou
(ii)

a1) déterminant la proportion du genre *Variovorax,* la proportion de bactéries appartenant au genre *Sphingomonas,* la proportion de bactéries appartenant au genre *Stenotrophomonas,* la proportion de bactéries appartenant au genre *Bosea,* et la proportion de bactéries appartenant au genre *Micrococcus,* par rapport au niveau bactérien total, et éventuellement le niveau bactérien total, dans un échantillon de sang ou de fèces du sujet avant le traitement médicamenteux, et
a2) combinant les proportions, et éventuellement le niveau bactérien total, déterminés à l'étape a1) afin d'obtenir une valeur de combinaison avant traitement ;
ou

(iii)

a1) déterminant la proportion du genre *Variovorax,* la proportion de bactéries appartenant au phylum des *Proteobacteria* et la proportion de bactéries appartenant au phylum des *Actineobacteria,* par rapport au niveau bactérien total, et éventuellement le niveau bactérien total, dans un échantillon de sang ou de fèces dudit sujet avant le traitement médicamenteux, et
a2) combinant les proportions, et éventuellement le niveau bactérien total, déterminés à l'étape a1) afin d'obtenir une valeur de combinaison avant traitement,

a') déterminer la diversité bactérienne dans un échantillon de sang ou de fèces du sujet après le traitement médicamenteux en

(i) déterminant l'indice de Shannon, ou
(ii)

a'1) déterminant la proportion du genre *Variovorax,* la proportion de bactéries appartenant au genre *Sphingomonas,* la proportion de bactéries appartenant au genre *Stenotrophomonas,* la proportion de bactéries appartenant au genre *Bosea,* et la proportion de bactéries appartenant au genre *Micrococcus,* par rapport au niveau bactérien total, et éventuellement le niveau bactérien total, dans un échantillon de sang ou de fèces du sujet après le traitement médicamenteux, et
a'2) combinant les proportions, et éventuellement le niveau bactérien total, déterminés à l'étape a1) afin d'obtenir une valeur de combinaison après traitement ;
ou

(iii)

a'1) déterminant la proportion du genre *Variovorax,* la proportion de bactéries appartenant au phylum des *Proteobacteria* et la proportion de bactéries appartenant au phylum des *Actineobacteria,* par rapport au niveau bactérien total, et éventuellement le niveau bactérien total, dans un échantillon de sang ou de fèces dudit sujet après le traitement médicamenteux, et
a'2) combinant les proportions, et éventuellement le niveau bactérien total, déterminés à l'étape a1) afin d'obtenir une valeur de combinaison après traitement,
et

b) en se basant sur le résultat des déterminations de la diversité bactérienne aux étapes a)(i) et a')(i) ou sur les valeurs de combinaison déterminées aux étapes (ii) a2) ou (iii) a2) et (ii) a'2) ou (iii) a'2), déterminer si le patient répond audit traitement médicamenteux.

**3.** Procédé pour cribler un probiotique, un prébiotique, un composé chimique ou un composé biologique approprié pour prévenir et/ou traiter la fibrose hépatique, comprenant les étapes consistant à :

A) déterminer la diversité bactérienne dans un échantillon de sang ou de fèces d'un sujet obèse souffrant de fibrose hépatique qui a été traité avec le probiotique, prébiotique, composé chimique ou biologique candidat, en

(i) déterminant l'indice de Shannon, ou
(ii)

A1) déterminant la proportion du genre *Variovorax,* la proportion de bactéries appartenant au genre *Sphingomonas,* la proportion de bactéries appartenant au genre *Stenotrophomonas,* la proportion de bactéries appartenant au genre *Bosea,* et la proportion de bactéries appartenant au genre *Micrococcus,* par rapport au niveau bactérien total, et éventuellement le niveau bactérien total, dans un échantillon de sang ou de fèces d'un sujet obèse souffrant de fibrose hépatique qui a été traité avec le probiotique, prébiotique, composé chimique ou biologique candidat, et
A2) combinant les proportions, et éventuellement le niveau bactérien total, déterminés à l'étape A1) afin d'obtenir une valeur de combinaison, ou

(iii)

A1) déterminant la proportion du genre *Variovorax,* la proportion de bactéries appartenant au phylum des *Proteobacteria* et la proportion de bactéries appartenant au phylum des *Actineobacteria,* par rapport au niveau bactérien total, et éventuellement le niveau bactérien total, dans un échantillon de sang ou de fèces d'un sujet obèse souffrant de fibrose hépatique qui a été traité avec le probiotique, prébiotique, composé chimique ou biologique candidat, et
A2) combinant les proportions, et éventuellement le niveau bactérien total, déterminés à l'étape A1) afin d'obtenir une valeur de combinaison
et

B) comparer ladite diversité bactérienne ou ladite valeur de combinaison avec celle d'un sujet obèse de contrôle souffrant de fibrose hépatique qui n'a pas été traité avec ledit probiotique, prébiotique, composé chimique ou composé biologique candidat, et
C) en se basant sur le résultat de la comparaison à l'étape B), déterminer si ledit probiotique, prébiotique, composé chimique ou composé biologique candidat est approprié pour prévenir et/ou traiter la fibrose hépatique.

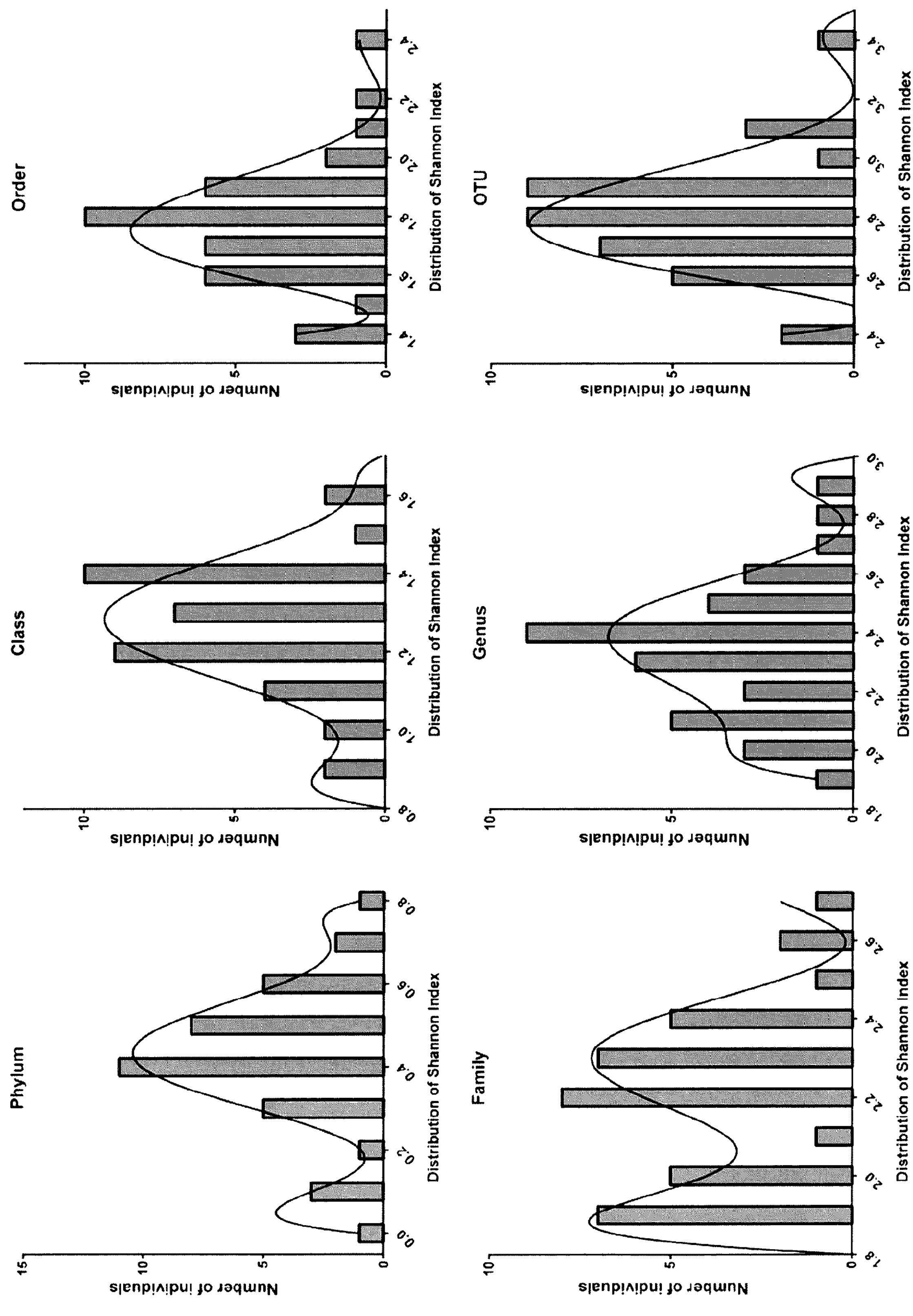
Phylum
Class
Order
Family
Genus
OTU
Number of individuals
Distribution of Shannon Index

FIG.1

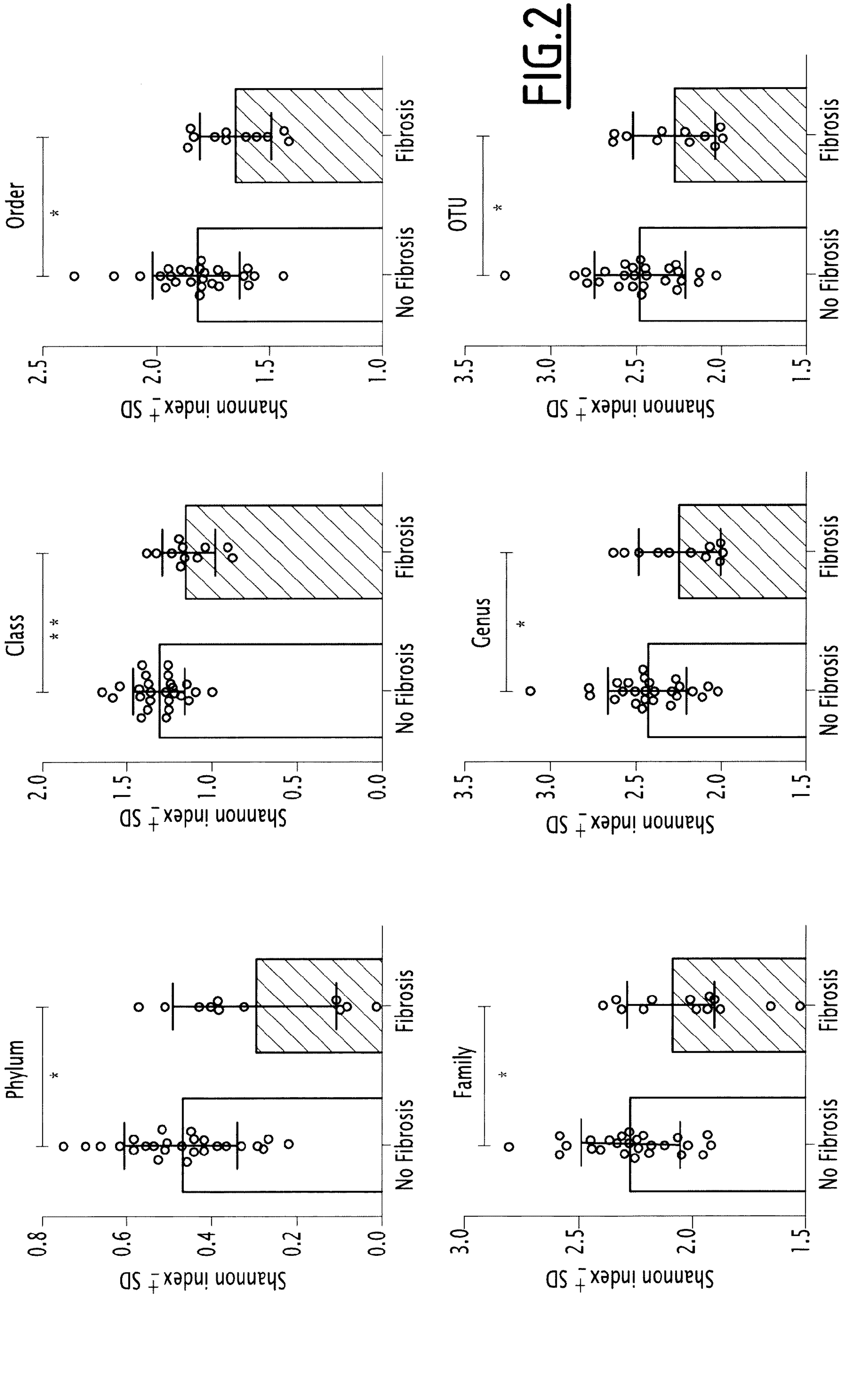

Phylum
Class
Order
Family
Genus
OTU
*
* *
Shannon index ± SD
No Fibrosis
Fibrosis
0.0
0.2
0.4
0.6
0.8
0.5
1.0
1.5
2.0
2.5
3.0
3.5


FIG.2

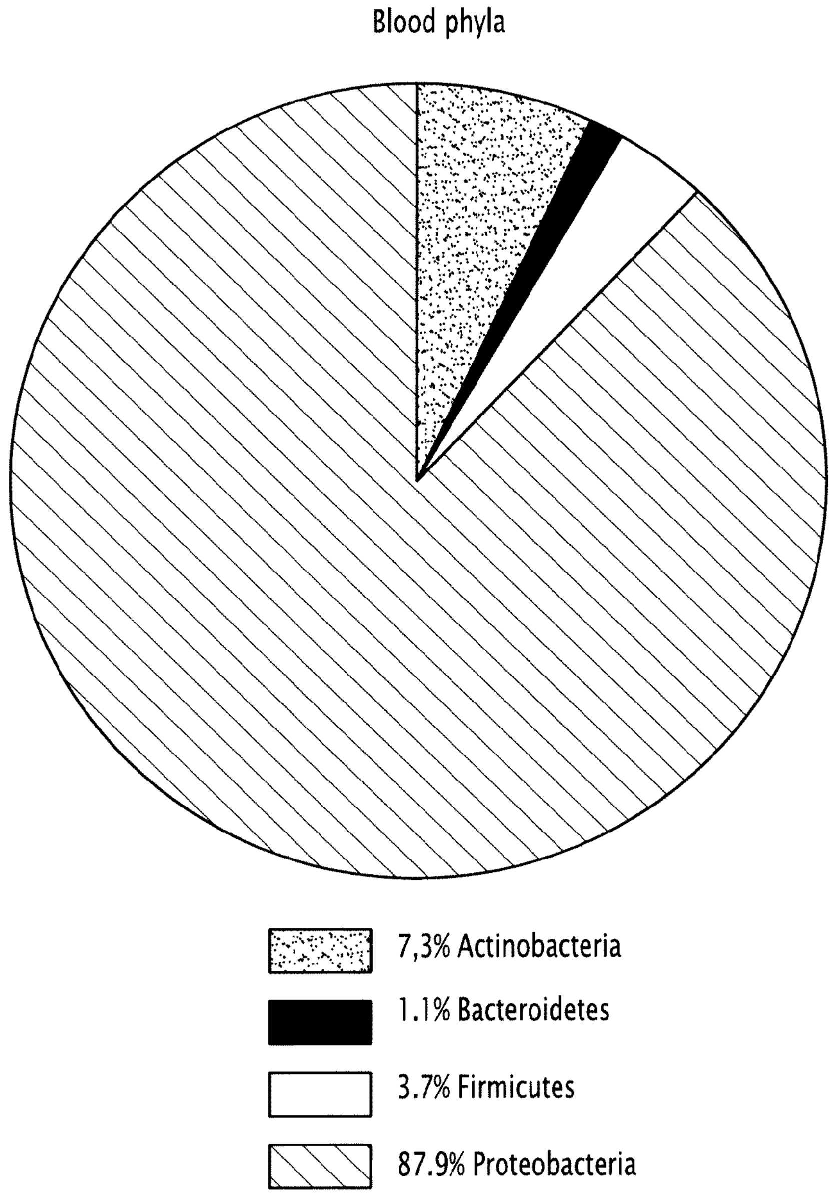
Blood phyla
7,3% Actinobacteria
1.1% Bacteroidetes
3.7% Firmicutes
87.9% Proteobacteria

FIG.3

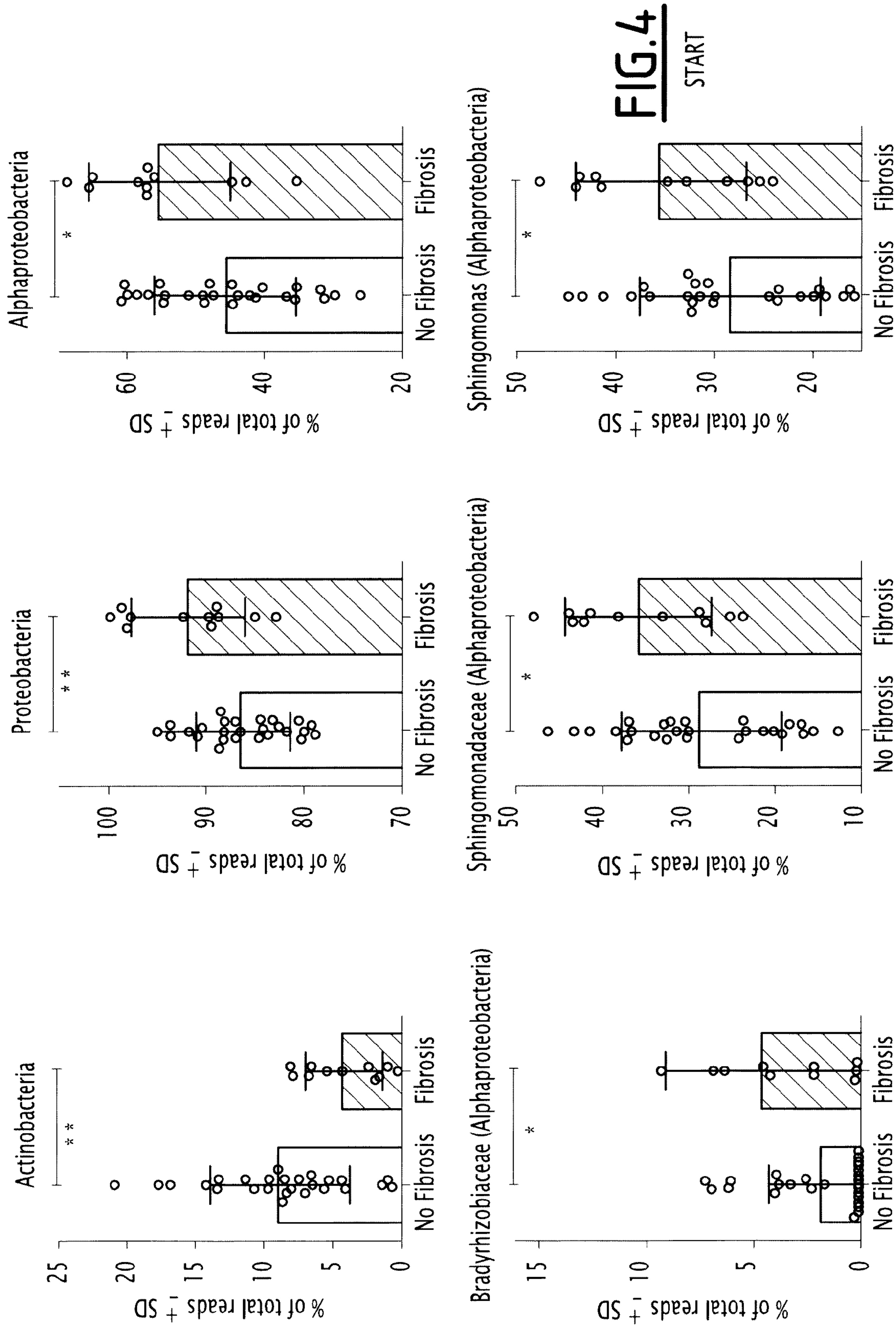
Actinobacteria
Proteobacteria
Alphaproteobacteria
Bradyrhizobiaceae (Alphaproteobacteria)
Sphingomonadaceae (Alphaproteobacteria)
Sphingomonas (Alphaproteobacteria)
% of total reads ± SD
No Fibrosis
Fibrosis
*
**

FIG.4
START

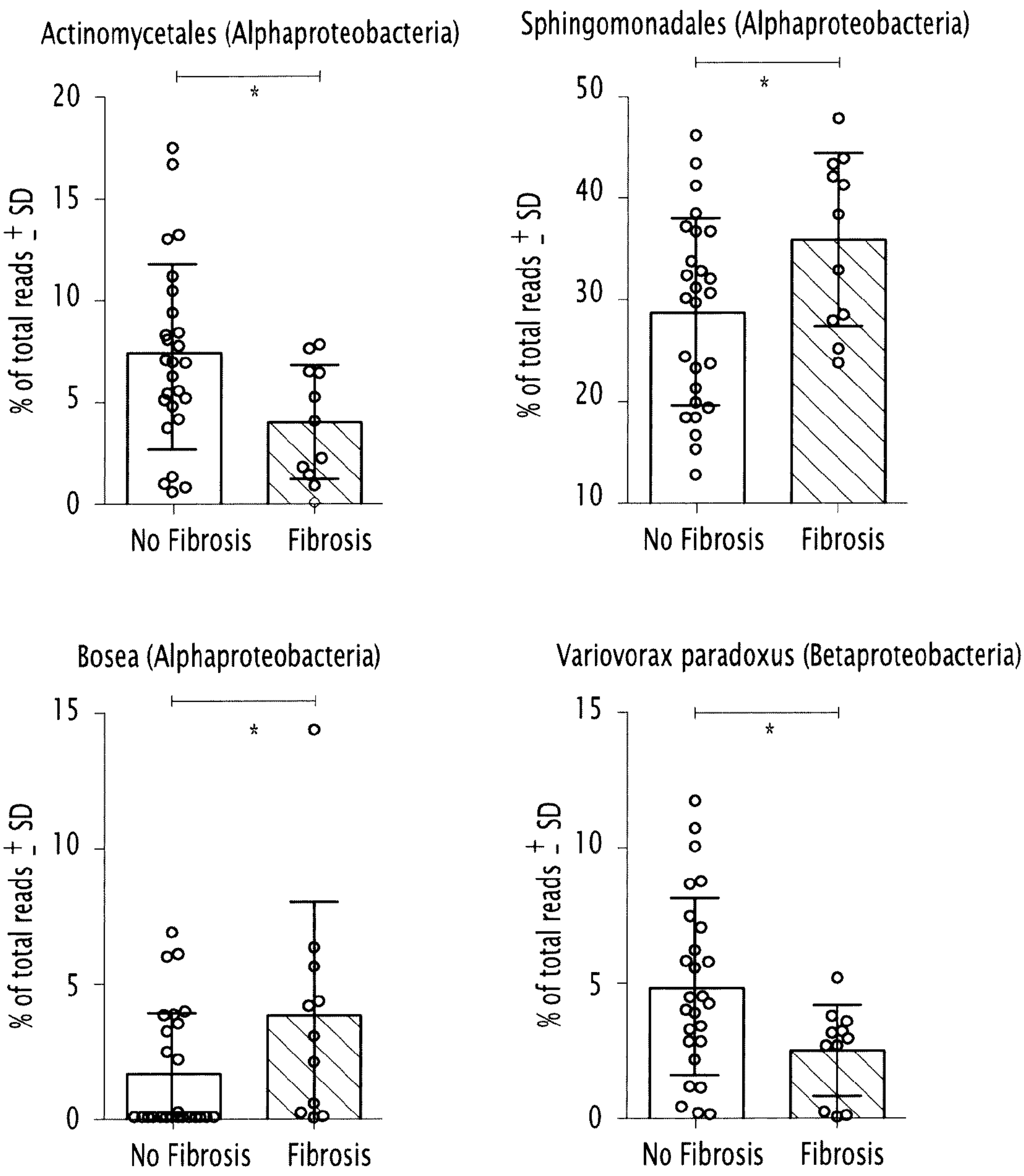
Actinomycetales (Alphaproteobacteria)
% of total reads ± SD
*
No Fibrosis
Fibrosis
Sphingomonadales (Alphaproteobacteria)
% of total reads ± SD
*
No Fibrosis
Fibrosis
Bosea (Alphaproteobacteria)
% of total reads ± SD
*
No Fibrosis
Fibrosis
Variovorax paradoxus (Betaproteobacteria)
% of total reads ± SD
*
No Fibrosis
Fibrosis

FIG.4

END

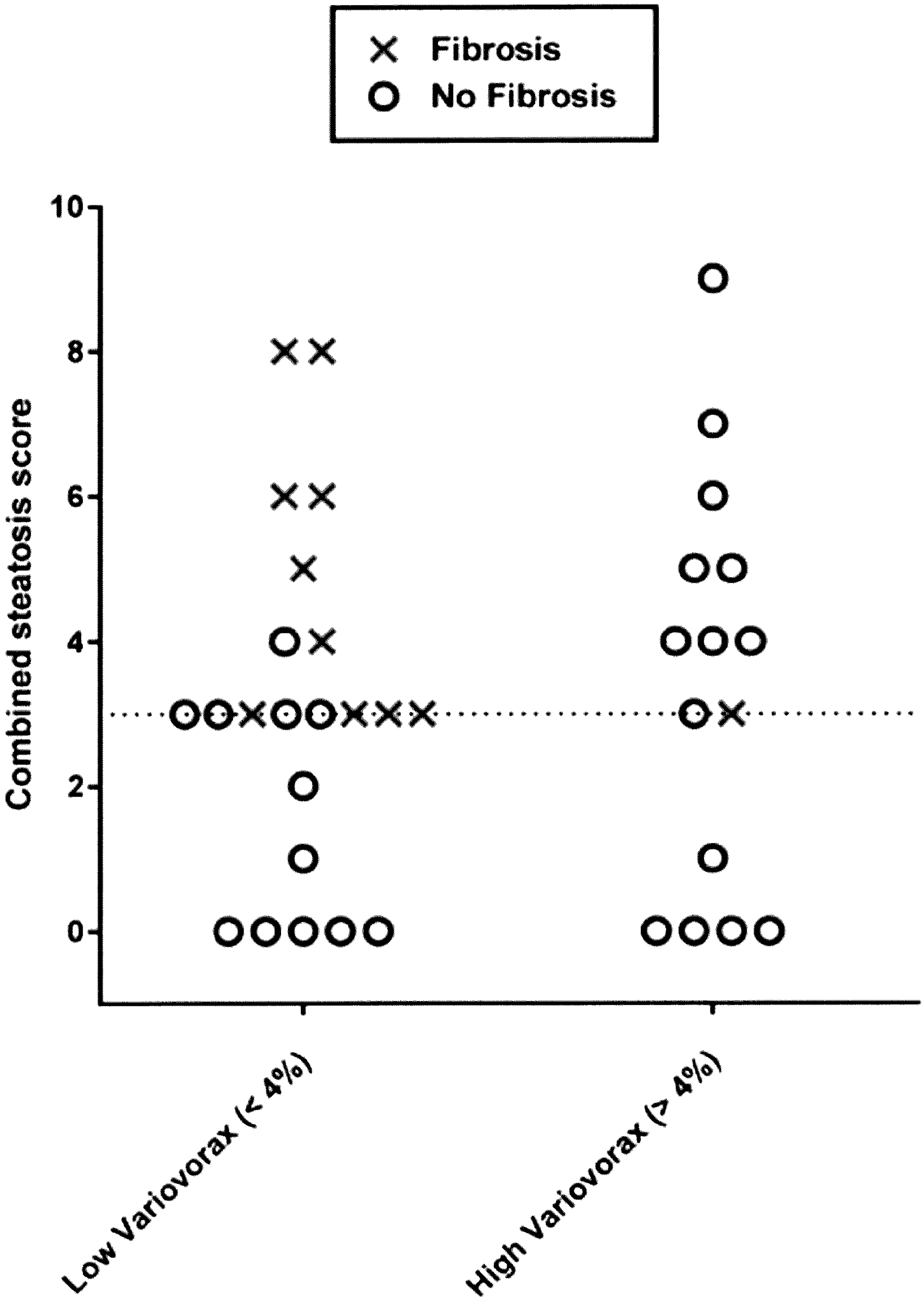
Fibrosis
No Fibrosis
10
8
6
4
2
0
Combined steatosis score
Low Variovorax (< 4%)
High Variovorax (> 4%)

FIG.5

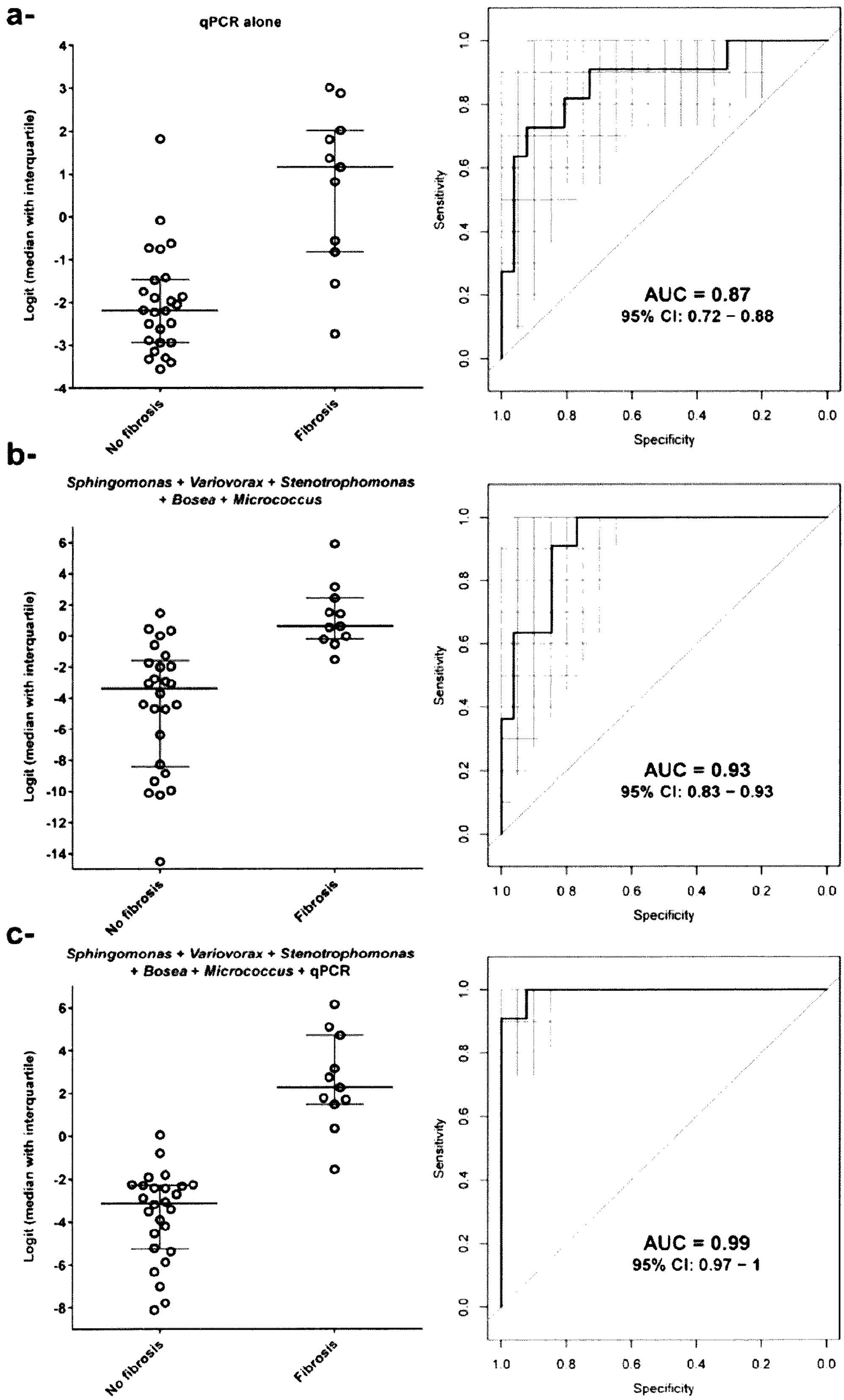
a-
qPCR alone
Logit (median with interquartile)
No fibrosis
Fibrosis
Sensitivity
Specificity
AUC = 0.87
95% CI: 0.72 – 0.88
b-
Sphingomonas + Variovorax + Stenotrophomonas + Bosea + Micrococcus
Logit (median with interquartile)
No fibrosis
Fibrosis
Sensitivity
Specificity
AUC = 0.93
95% CI: 0.83 – 0.93
c-
Sphingomonas + Variovorax + Stenotrophomonas + Bosea + Micrococcus + qPCR
Logit (median with interquartile)
No fibrosis
Fibrosis
Sensitivity
Specificity
AUC = 0.99
95% CI: 0.97 – 1

FIG.6 START

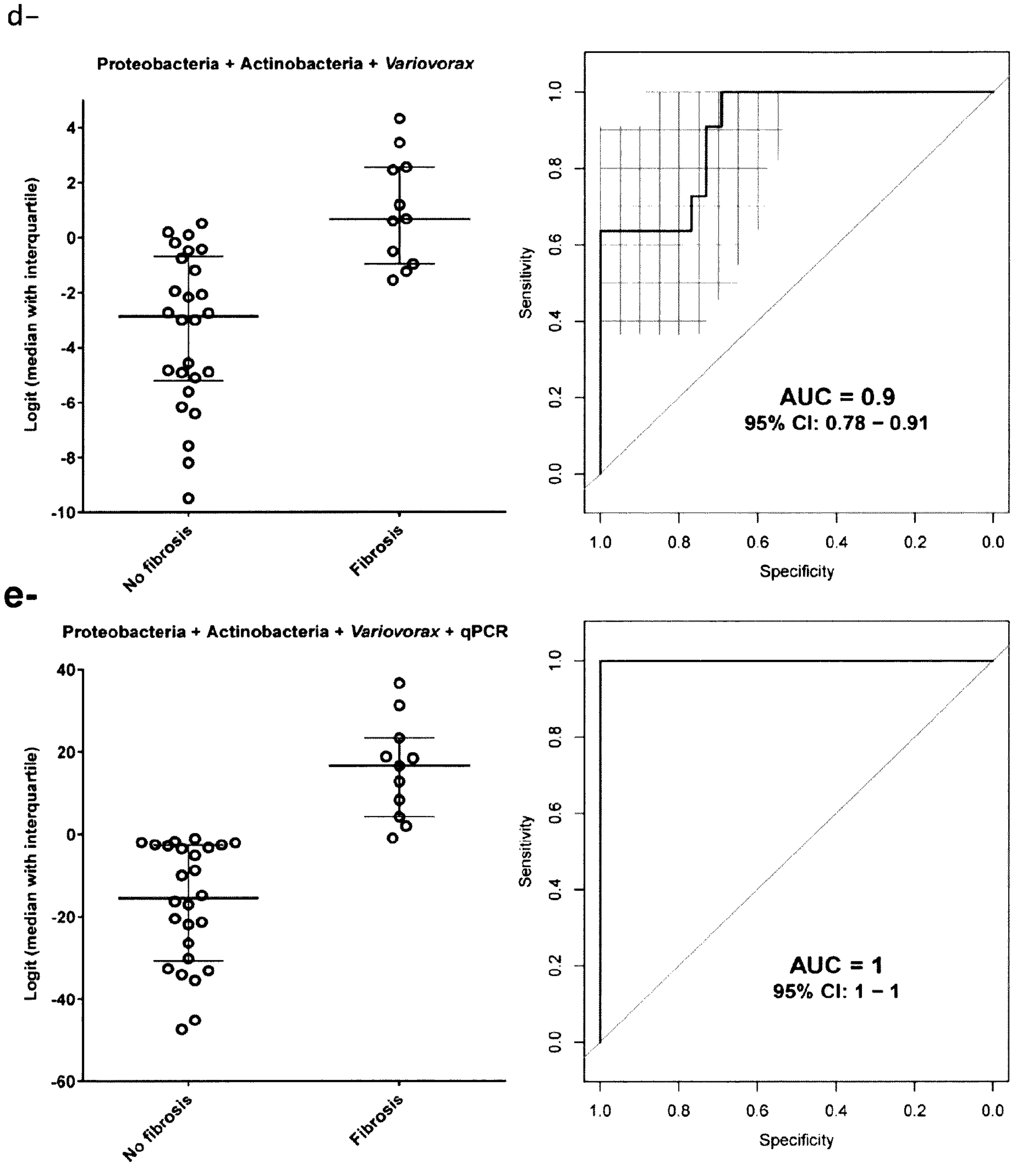
d-
Proteobacteria + Actinobacteria + *Variovorax*
Logit (median with interquartile)
4
2
0
-2
-4
-6
-8
-10
No fibrosis
Fibrosis
1.0
0.8
0.6
0.4
0.2
0.0
Sensitivity
Specificity
AUC = 0.9
95% CI: 0.78 – 0.91
e-
Proteobacteria + Actinobacteria + *Variovorax* + qPCR
40
20
0
-20
-40
-60
AUC = 1
95% CI: 1 – 1

FIG.6

END

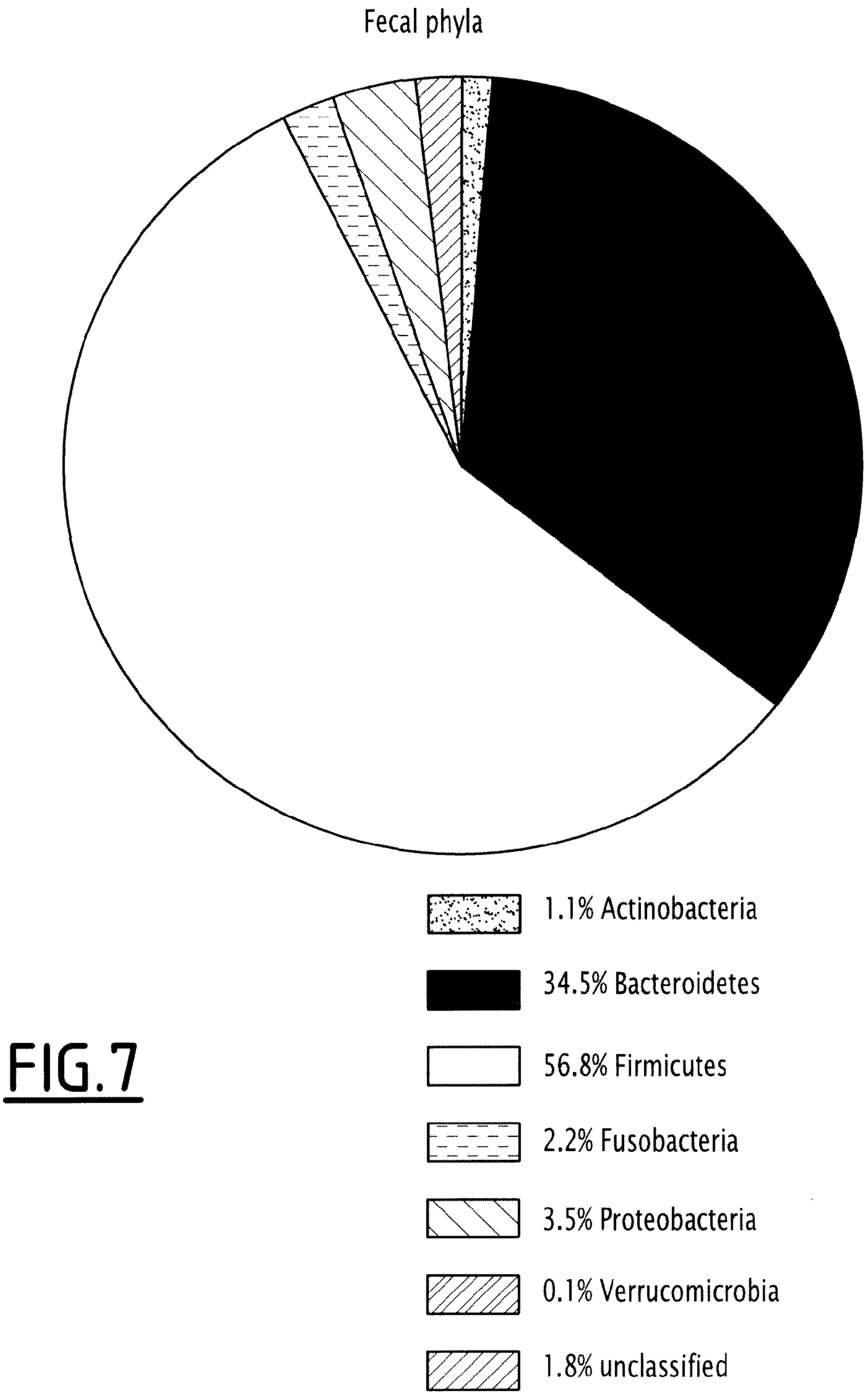
Fecal phyla
1.1% Actinobacteria
34.5% Bacteroidetes
56.8% Firmicutes
2.2% Fusobacteria
3.5% Proteobacteria
0.1% Verrucomicrobia
1.8% unclassified

FIG.7

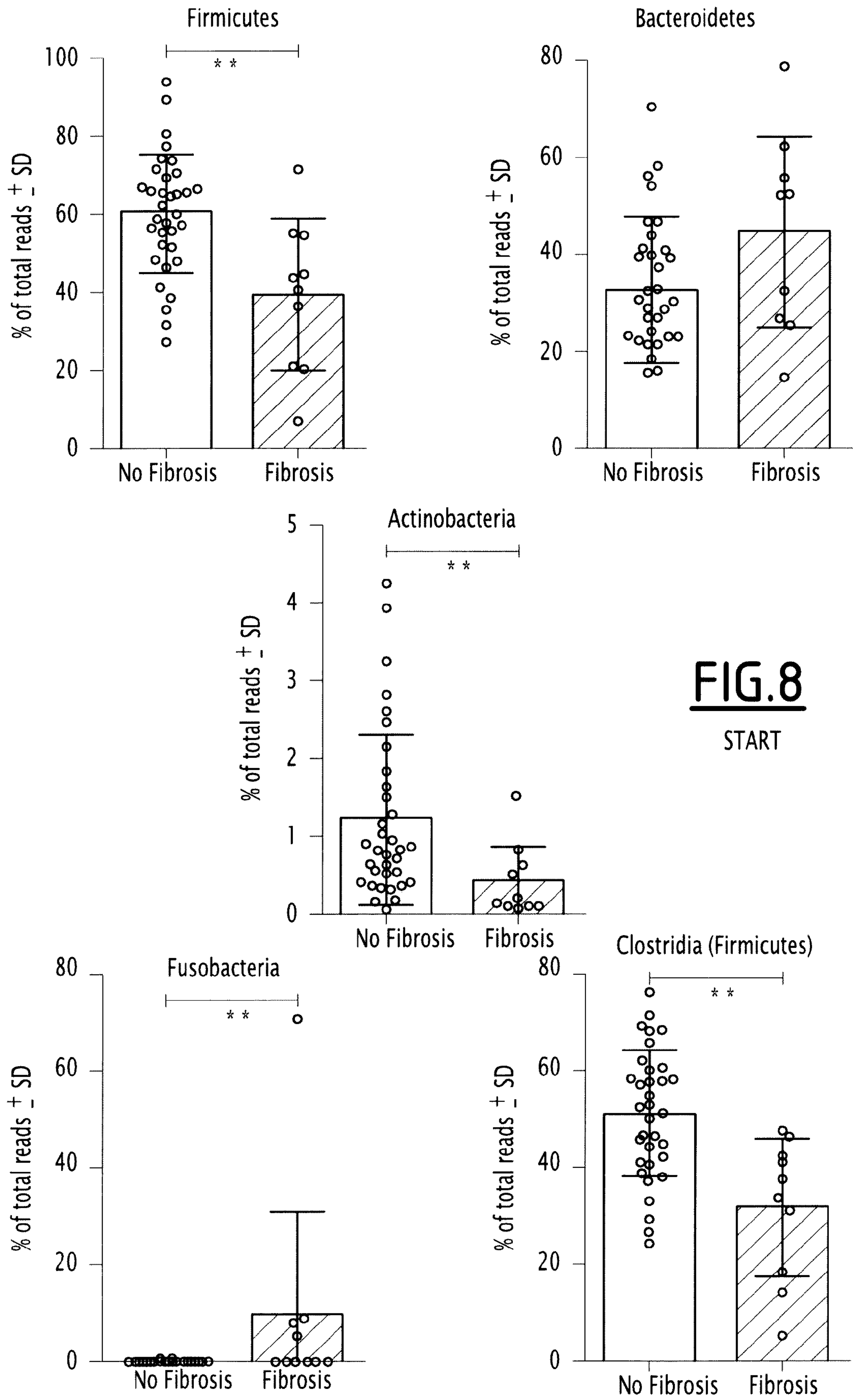
Firmicutes
Bacteroidetes
Actinobacteria
Fusobacteria
Clostridia (Firmicutes)
% of total reads + _ SD
No Fibrosis
Fibrosis
* *
100
80
60
40
20
0
5
4
3
2
1

FIG.8

START

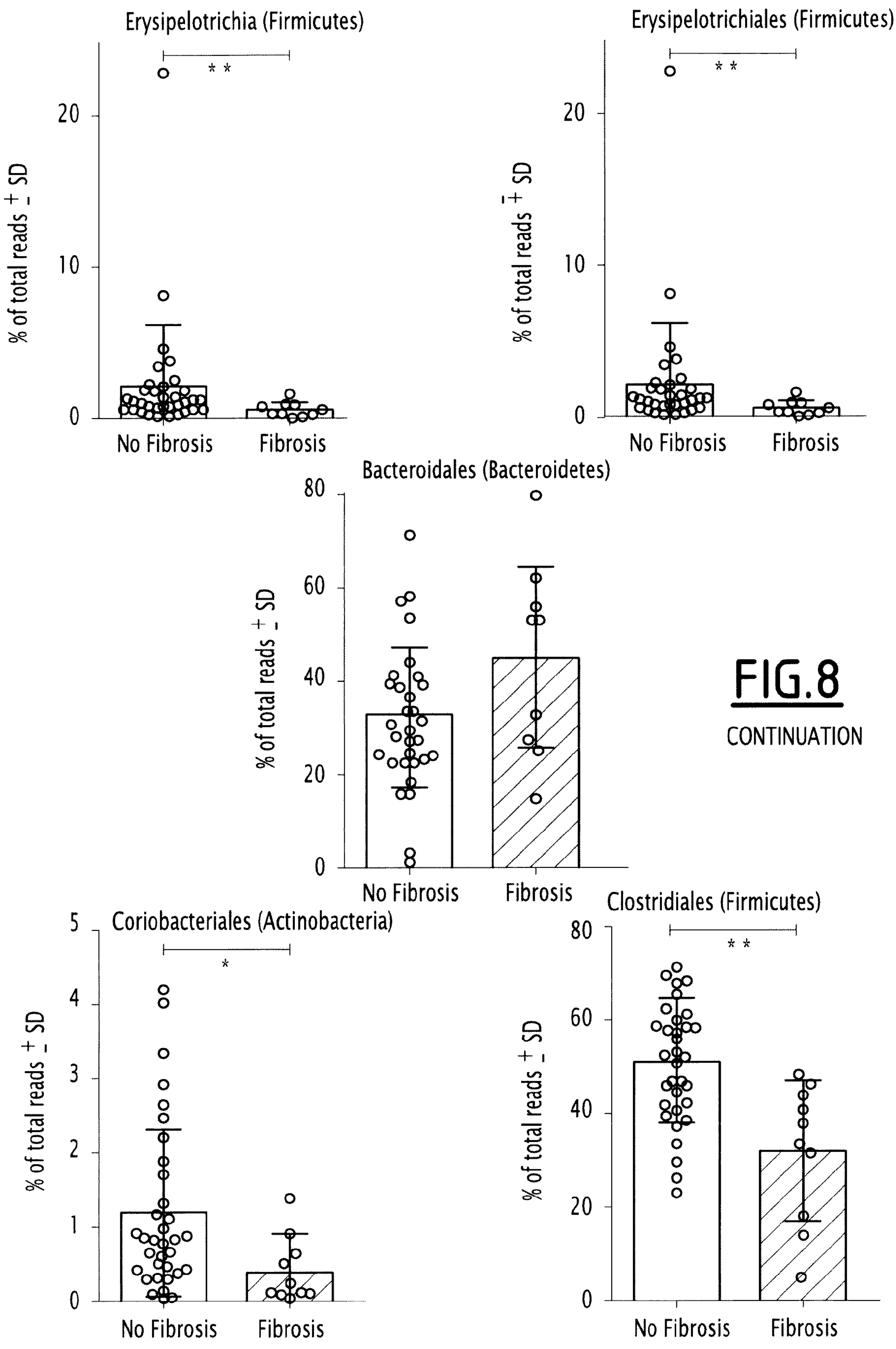


FIG.8
CONTINUATION

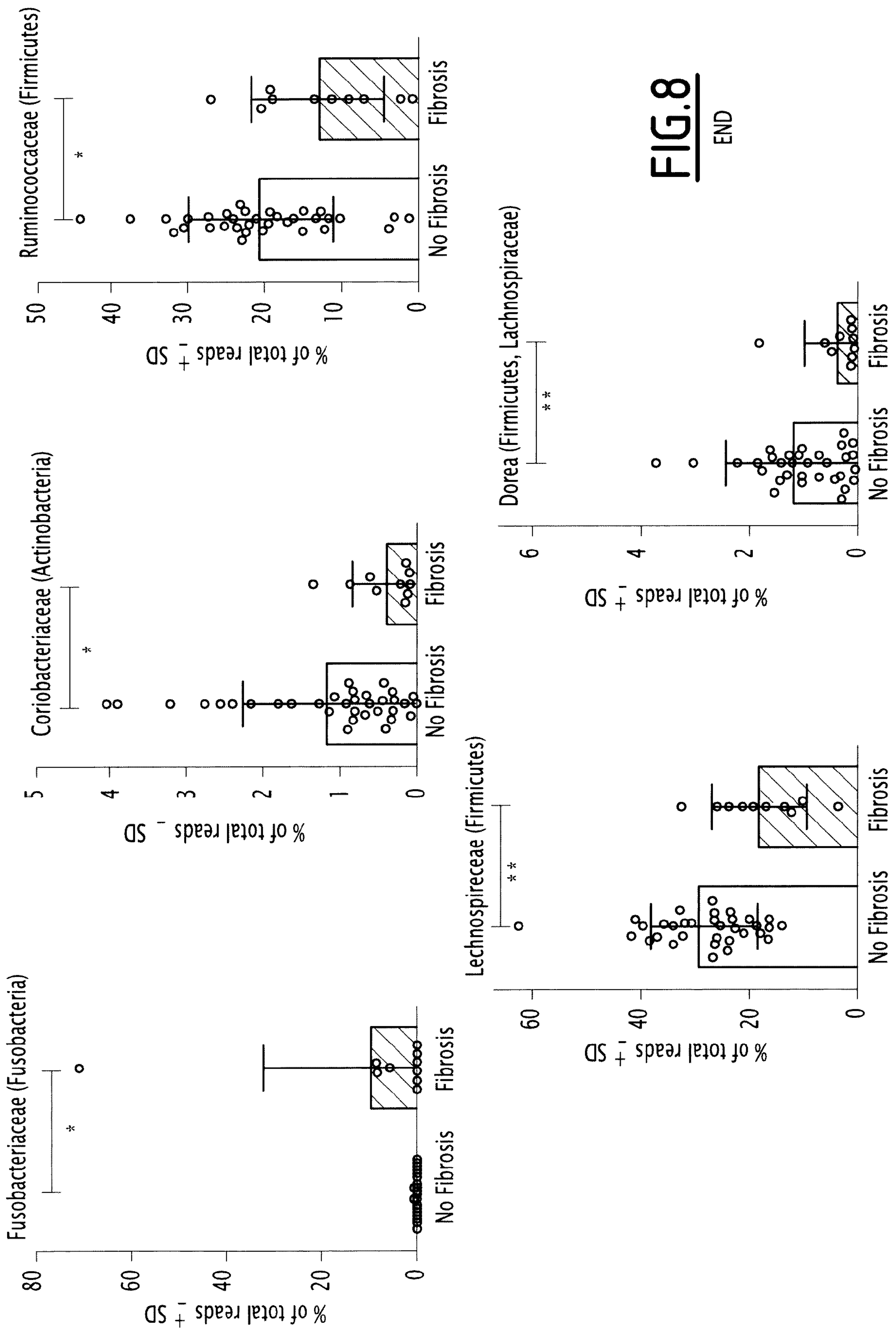
Ruminococcaceae (Firmicutes)
Coriobacteriaceae (Actinobacteria)
Fusobacteriaceae (Fusobacteria)
Lechnospireceae (Firmicutes)
Dorea (Firmicutes, Lachnospiraceae)
% of total reads ± SD
No Fibrosis
Fibrosis
*
**

FIG.8
END

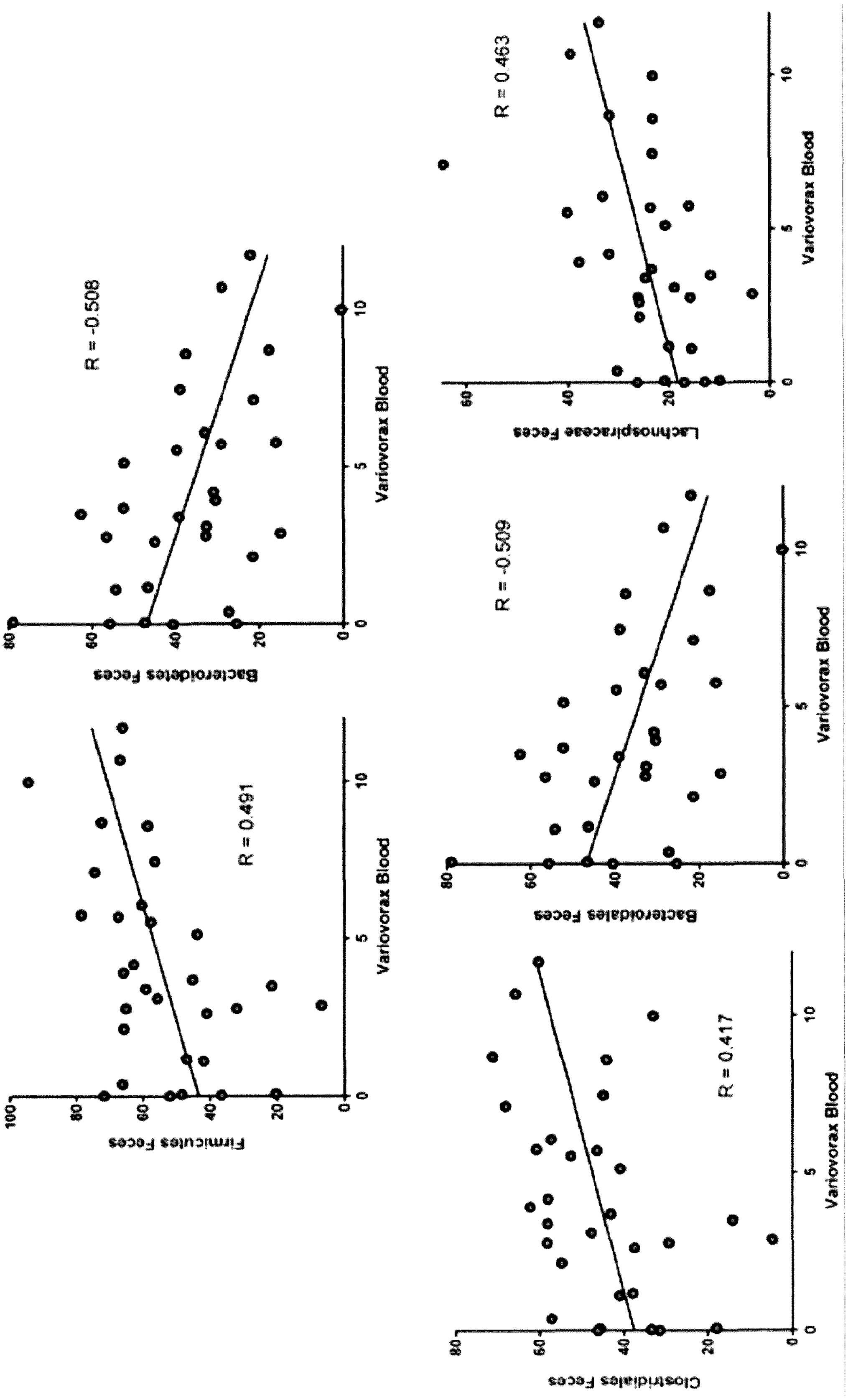
Firmicutes Feces
R = 0.491
Variovorax Blood
Bacteroidetes Feces
R = -0.508
Variovorax Blood
Clostridiales Feces
R = 0.417
Variovorax Blood
Bacteroidales Feces
R = -0.509
Variovorax Blood
Lachnospiraceae Feces
R = 0.463
Variovorax Blood

FIG.9

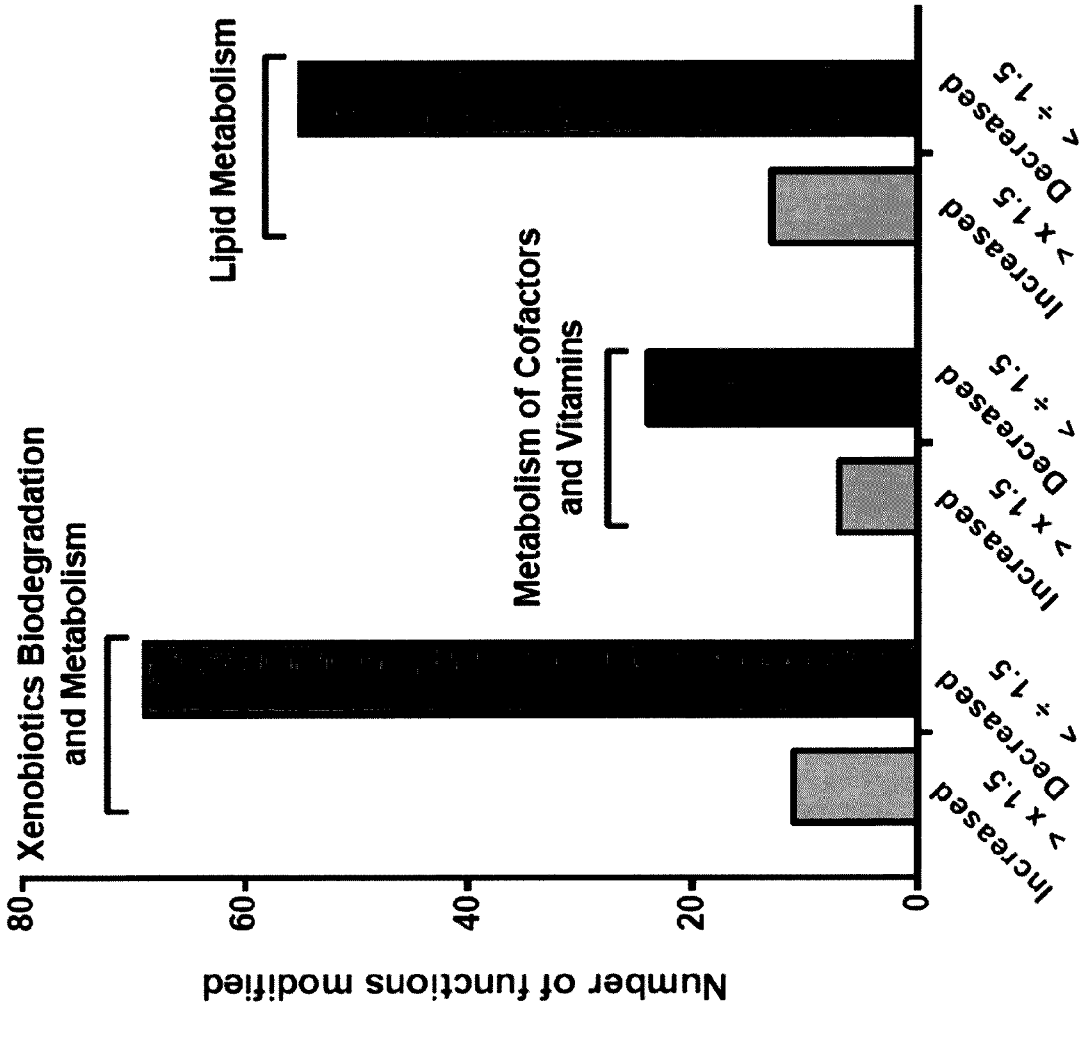
Xenobiotics Biodegradation and Metabolism
Metabolism of Cofactors and Vitamins
Lipid Metabolism
Number of functions modified
0
20
40
60
80
Increased > x 1.5
Decreased > ÷ 1.5
Increased > x 1.5
Decreased > ÷ 1.5
Increased > x 1.5
Decreased > ÷ 1.5

FIG.10

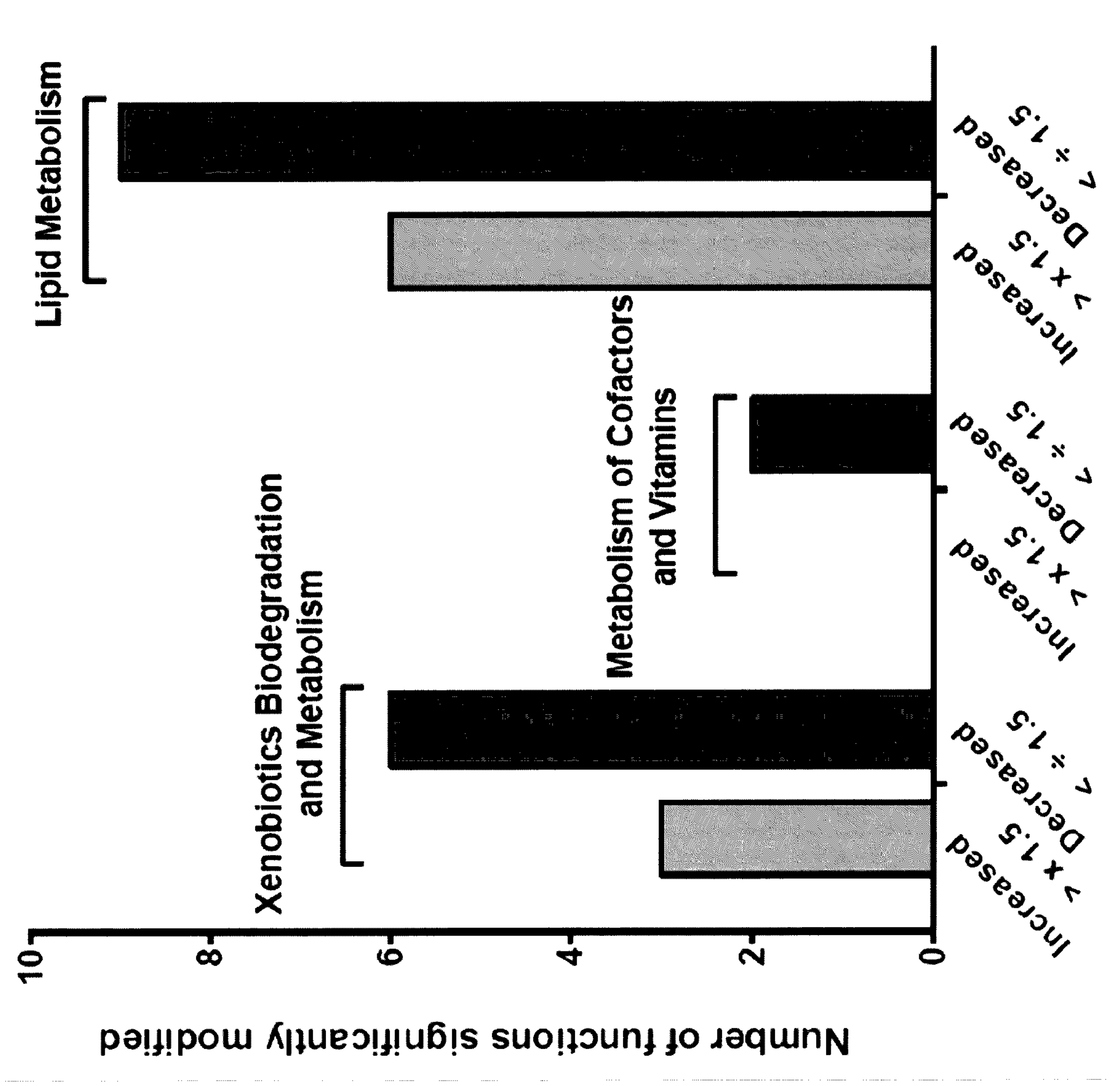
Number of functions significantly modified
0
2
4
6
8
10
Xenobiotics Biodegradation and Metabolism
Metabolism of Cofactors and Vitamins
Lipid Metabolism
Increased > x 1,5
Decreased > ÷ 1,5
Increased > x 1,5
Decreased > ÷ 1,5
Increased > x 1,5
Decreased > ÷ 1,5

FIG.11

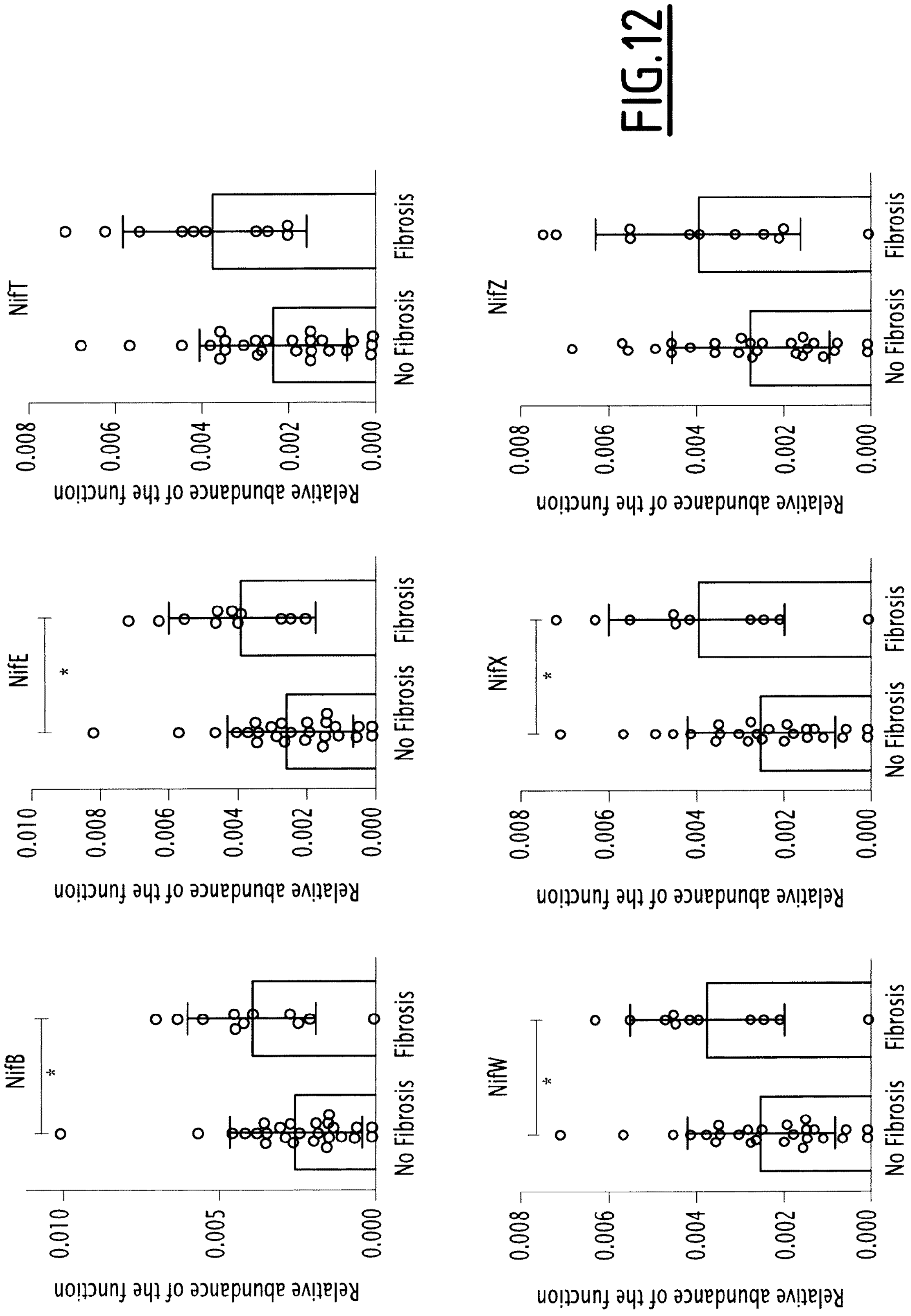
NifB
NifE
NifT
NifW
NifX
NifZ
*
Relative abundance of the function
0.010
0.005
0.008
0.006
0.004
0.002
0.000
No Fibrosis
Fibrosis

FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- UA 77798
- UA 77726
- EP 15306606

### Non-patent literature cited in the description


- **LANGILLE et al.** *Nat. Biotechnol.,* 2013, vol. 31, 814-821
- **AβHAUER et al.** *Bioinformatics,* 2015, vol. 31, 2882-2884
- **BOWMAN et al.** *PLoS ONE,* 2015, vol. 10, e0135868
- **KOZICH et al.** *Appl. Environ. Microbiol,* 2013, vol. 79, 5112-5120
- **SERINO et al.** *Gut,* 2012, vol. 61, 543-553
- **SEGATA et al.** *Genome Biology,* 2011, vol. 12, R60
- **LANGILLE et al.** *Nat. Biotechnol.,* 2013, vol. 31, 814-82